# EUROPÄISCHE PATENTSCHRIFT

(11) **EP 1 068 223 B1**
(45) Veröffentlichungstag und Bekanntmachung des Hinweises auf die Patenterteilung: **04.09.2002**
(21) Anmeldenummer: 99907547.6
(22) Anmeldetag: 12.02.1999
(51) Int. Cl.: C07K 7/60, A61K 38/12

(54) **LIPOPEPTIDANTIBIOTIKA-CALCIUMSALZE, VERFAHREN ZU IHRER HERSTELLUNG UND VERWENDUNG**
CALCIUM SALTS OF LIPOPEPTIDE ANTIBIOTICS, METHOD FOR PRODUCING SAME AND THEIR USE
SELS DE CALCIUM D'ANTIBIOTIQUES A BASE DE LIPOPEPTIDES, LEUR PROCEDE DE PRODUCTION ET LEUR UTILISATION

(30) Priorität: 25.02.1998 DE 19807972
(43) Veröffentlichungstag der Anmeldung: 17.01.2001
(73) Patentinhaber: Aventis Pharma Deutschland GmbH, 65929 Frankfurt am Main (DE)
(72) Erfinder: VERTESY, Laszlo, D-65817 Eppstein (DE); ARETZ, Werner, D-61462 Königstein (DE); DECKER, Heinrich, D-65817 Bremtal (DE); EHLERS, Eberhard, D-65719 Hofheim (DE); KURZ, Michael, D-65719 Hofheim (DE); SCHMIDT, Frank, Rainer, D-65719 Hofheim (DE)
(86) Internationale Anmeldenummer: EP9900930
(87) Internationale Veröffentlichungsnummer: WO99043700

(56) Entgegenhaltungen:
- EP-A1- 0 629 636
- US-A- 5 629 288

## Beschreibung

Die vorliegende Erfindung betrifft Calciumsalze von Lipopeptidantibiotika, ein Verfahren zu ihrer Herstellung und ihre Verwendung.

Aus der EP 0 629 636 A1 sind Lipopeptidantibiotika der allgemeinen Formel I bekannt, in der R¹ eine OH- oder eine NH₂-Gruppe und R² einen Fettsäurerest (R-C(O)-) bedeuten.

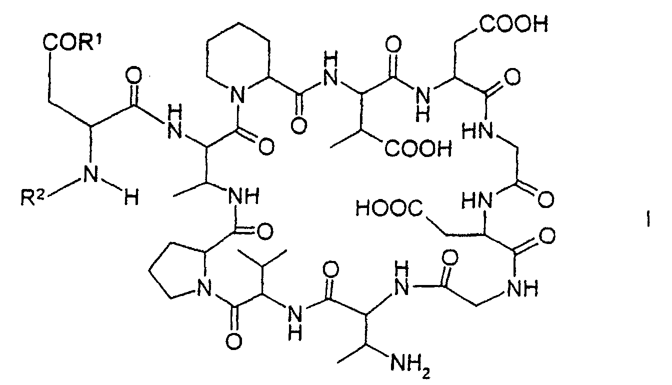

Diese Lipopeptidantibiotika lassen sich in zwei Gruppen unterteilen, welche sich in bezug auf ihre exozyklische Aminosäure unterscheiden: Die Lipopeptidantibiotika vom Amphomycin-Typ sind durch die exozyklische Aminosäure Asparaginsäure (Asp, wobei R¹ in Formel I eine OH-Gruppe bedeutet) gekennzeichnet (R. C. Strong et al., Antimicrobial Agents and Chemotherapy 1970, 42-45; M. Bodanszky et al. J. Am. Chem. Soc. 95, 2352-2357 (1973)), während sich die Lipopeptidantibiotika vom Asparagin-Typ durch die exozyklische Aminosäure Asparagin (Asn, wobei R¹ in Formel I eine NH₂-Gruppe bedeutet) auszeichnen. Untereinander unterscheiden sich die Lipopeptidantibiotika vom Amphomycin- und vom Asparagin-Typ durch Substitution an der α-Aminogruppe der exozyklischen Aminosäure (Asp oder Asn) mit unterschiedlichen Fettsäureresten (R² in Formel I).

Ferner sind aus der EP 0 688 789 A1 (US 5,629,288) Derivate der Lipopeptidantibiotika vom Amphomycin- und vom Asparagin-Typ und deren pharmazeutisch verträgliche Salze bekannt. Als pharmazeutisch verträgliche Salze der Lipopeptidantibiotika der Formel I werden in der EP 0 688 789 A1 (US 5,629,288) Salze mit anorganischen und organischen Säuren, z.B. Salzsäure, Schwefelsäure, Essigsäure, Citronensäure, p-Toluolsulfonsäure, mit anorganischen und organischen Basen wie NaOH, KOH, Mg(OH)₂, Diethanolamin, Ethylendiamin oder mit Aminosäuren wie Arginin, Lysin und Glutaminsäure offenbart.

Ferner ist das Calciumsalz von Amphomycin bekannt (Kirk-Othmer, Encyclopedia of Chemical Technology, vierte Auflage, Band 3, Antibiotics to Batteries, John Wiley & Sons, Seite 284). Es ist in Wasser nur schwer löslich und wurde wegen der Toxizität von Amphomycin infolge dessen hämolytischen Aktivität bei systemischer Anwendung ausschließlich in antibiotischen Salben zur lokalen Applikation verwendet.

Die Lipopeptidantibiotika vom Asparagin-Typ (R¹ in Formel I bedeutet eine NH₂-Gruppe) und deren Herstellung sind erstmals in der EP 0 629 636 A1 beschrieben worden. Das dort vorgeschlagene Herstellungsverfahren, die Fermentation von Actinoplanes sp., vorzugsweise Actinoplanes friulensis (am 18. Juni 1990 gemäß den Vorschriften des Budapester Vertrags unter der Hinterlegungsnummer DSM 7358 bei der Deutschen Sammlung von Mikroorganismen und Zellkulturen GmbH, DSMZ, Mascheroder Weg 1b, D-38124 Braunschweig hinterlegt), führt jedoch zu einem Gemisch einer Vielzahl der strukturell nahe verwandten Lipopeptide vom Amphomycinund vom Asparagin-Typ, die sehr unterschiedliche Eigenschaften, insbesondere unterschiedliche biologische Wirkungen, wie zum Beispiel deren antibakterielle Wirkung, Toxizität infolge deren hämolytischen Wirkung, sowie unterschiedliche physikalisch-chemischen Eigenschaften, wie zum Beispiel deren Löslichkeit und Stabilität, aufweisen, aber aus dem Kulturmedium nur aufwendig getrennt werden können. Es wäre deshalb von großem Vorteil, über ein Fermentationsverfahren zu verfügen, welches im wesentlichen zur Produktion bevorzugt nur einer der vielen möglichen Lipopeptid-Komponenten führt.

Aufgabe der vorliegenden Erfindung ist es, ein Salz der Lipopeptidantibiotika vom Asparagin-Typ bereitzustellen, welches sich durch eine relativ hohe Stabilität sowie gute antibakterielle Wirksamkeit auszeichnet und infolge seiner guten Wasserlöslichkeit sowie infolge seiner geringen Toxizität, insbesondere aufgrund einer geringen hämolytischen Aktivität, systemisch (parenteral) anwendbar ist.

Ferner ist es Aufgabe der vorliegenden Erfindung, ein Verfahren zur Herstellung eines Salzes der Lipopeptidantibiotika vom Asparagin-Typ bereitzustellen und im besonderen ein verbessertes Verfahren zur fermentativen Herstellung dessen Säurevorstufe, bei dem bevorzugt Lipopeptidantibiotika vom Asparagin-Typ erzeugt werden, bereitzustellen.

Schließlich ist es Aufgabe der vorliegenden Erfindung, ein Arzneimittel bereitzustellen, das ein Salz der Lipopeptidantibiotika vom Asparagin-Typ mit den gewünschten vorteilhaften Eigenschaften enthält.

Es wurde nun bei den Lipopeptidantibiotika vom Asparagin-Typ gefunden, daß die verschiedenen Salze desselben Lipopeptids (bzw. derselben korrespondierenden Säure) sehr unterschiedliche Eigenschaften aufweisen können. Beispielsweise besitzen die Natriumsalze in der Regel eine sehr gute antibakterielle Wirksamkeit und sind in Wasser leicht löslich. Diese sind jedoch insbesondere bei erhöhten Temperaturen nur begrenzt haltbar. Da bei Medikamenten und anderen Handelsprodukten eine Stabilität z. B für die Handhabung der Ware von großer Wichtigkeit ist, sind stabile Salz-Formen der Lipopeptidantibiotika erforderlich.

Da die Lipopeptidantibiotika vom Asparagin-Typ amphotäre Verbindungen mit einem isoelektrischen Punkt im sauren Bereich sind, lassen sich neutrale Salze mit zahlreichen Basen herstellen. Als Kationen kommen einwertige und mehrwertige Ionen in Frage, wie zum Beispiel Alkali-, Erdalkali-, und andere Metallionen aber auch Salze mit Ammoniak oder mit organischen Basen, wie Aminen. Als Beispiel für letztere dienen Lysin- und Lysyllysin-Salze, die bei voller Wirksamkeit sehr gut verträglich sind.

Überraschend ist nun gefunden worden, daß im Gegensatz zu den Calciumsalzen der Lipopeptidantibiotika vom Amphomycin-Typ (insbesondere Amphomycin) die CalciumSalze der Lipopeptidantibiotika vom Asparagin-Typ nicht nur wirksam und verträglich, sondern auch in Wasser gut löslich und im Gegensatz zu den entsprechenden Natriumsalzen besonders beständig sind.

Dementsprechend wird die vorstehend gestellte Aufgabe gelöst durch ein Calciumsalz der Verbindung der Formel II 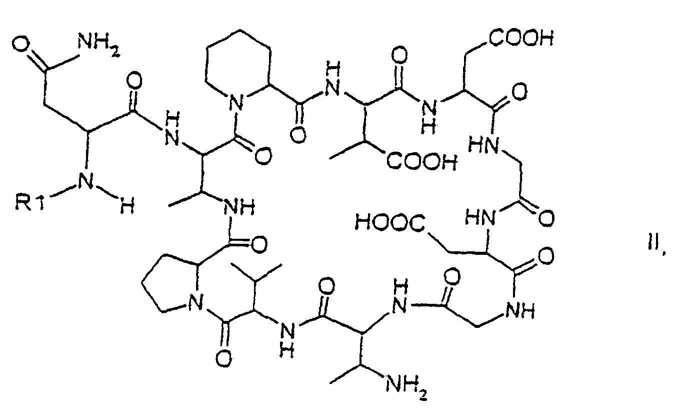 worin R1 ein geradkettiger oder verzweigter, gesättigter oder ungesättigter aliphatischer Acylrest mit 8 bis 22 Kohlenstoffatomen bedeutet, der wahlweise durch ein oder mehrere Phenyl- oder Cycloalkylgruppen unterbrochen oder mit solchen Gruppen verknüpft und ferner wahlweise durch Sauerstoff unterbrochen sein kann.

Vorzugsweise bedeutet R1 in Formel II ein durch eine Phenyl- oder Cycloalkylgruppe unterbrochener oder mit einer solchen Gruppe verknüpfter Acylrest, beispielsweise 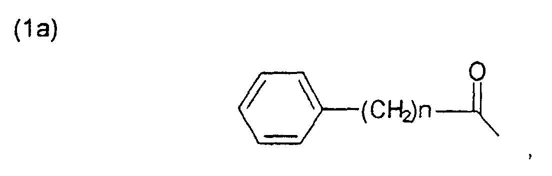 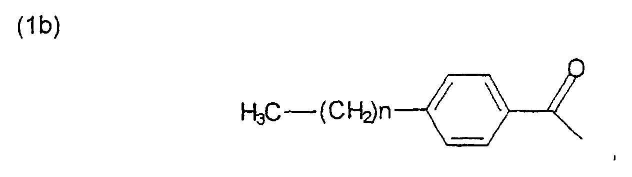 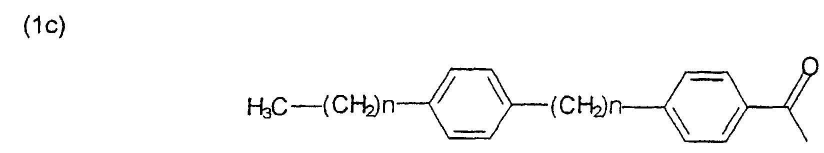 oder 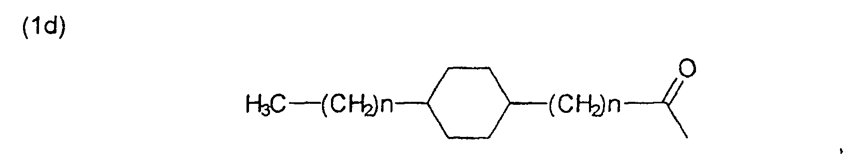 wobei n eine ganze Zahl von 0 bis 20 ist.

Ferner bevorzugt ist ein Calciumsalz der Verbindung der Formel II, das sich dadurch auszeichnet, daß R1 ein durch eine Phenyl- oder Cycloalkylgruppe und durch Sauerstoff unterbrochener Acylrest bedeutet, vorzugsweise dadurch gekennzeichnet, daß R1 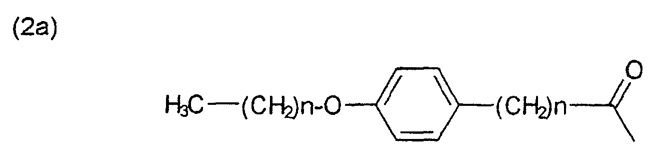 oder 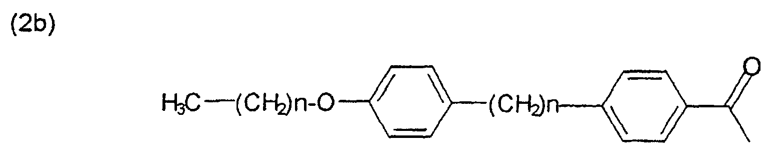 bedeutet, wobei n eine ganze Zahl von 0 bis 20 ist.

Besonders bevorzugt ist ein Calciumsalz der Verbindung der Formel II, welches sich dadurch auszeichnet, daß R1 ein geradkettiger oder verzweigter, gesättigter oder ungesättigter aliphatischer Acylrest mit 12 bis 15 Kohlenstoffatomen bedeutet, wobei R1 in Formel II vorzugsweise ein Fettsäurerest der nachfolgend dargestellten Formeln bedeutet: 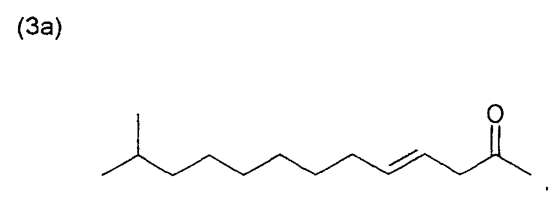 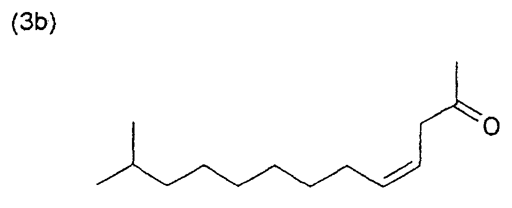 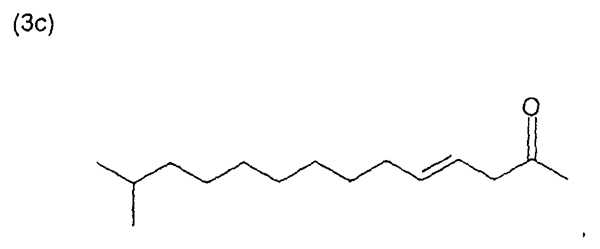 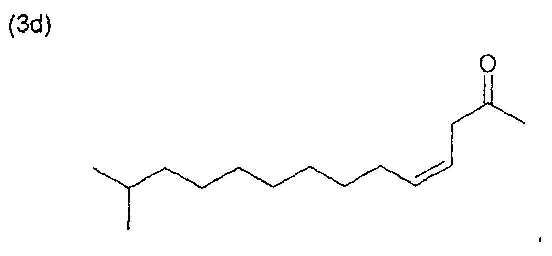 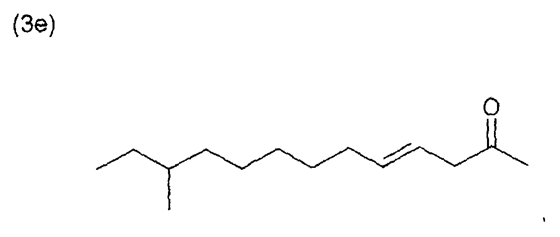 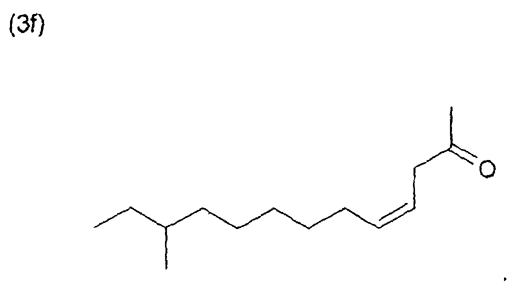 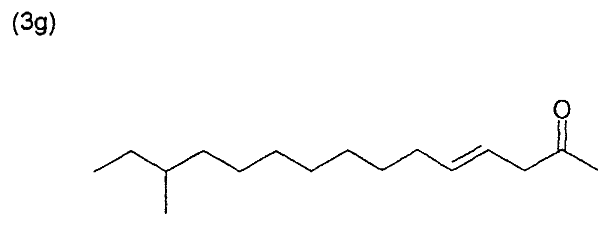 oder

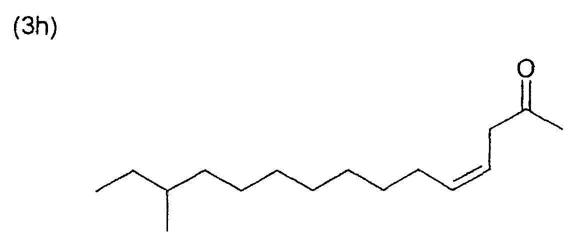

Das Calciumsalz der Verbindung der Formel II kann in zwei Formen vorliegen, als Dicalciumsalz oder als Monocalciumsalz.

Das Dicalciumsalz kann in Abhängigkeit von der Zahl der Anionen und dem Fettsäuresubstituenten (R1 in Formel II)
(i) beispielsweise im Fall einer gesättigten Fettsäure (R1 in Formel II) durch die allgemeine Summenformel
   (ia) C₄₆₊ₙH₆₈₊₂ₙN₁₄O₁₉Ca₂X₂,
   (ib) C₄₆₊ₙH₆₉₊₂ₙN₁₄O₁₉Ca₂X₃ oder
   (ic) C₄₆₊ₙH₇₀₊₂ₙN₁₄O₁₉Ca₂X₄,
   wobei in der allgemeinen Summenformel n eine ganze Zahl von 7 bis 21 und X ein Anion bedeuten, oder
(ii) beispielsweise im Fall einer einfach ungesättigten Fettsäure (R1 in Formel II) durch die allgemeine Summenformel
   (iia) C₄₆₊ₙH₆₆₊₂ₙN₁₄O₁₉Ca₂X₂
   (iib) C₄₆₊ₙH₆₇₊₂ₙN₁₄O₁₉Ca₂X₃ oder
   (iic) C₄₆₊ₙH₆₈₊₂ₙN₁₄O₁₉Ca₂X₄,
   wobei in der allgemeinen Summenformel n eine ganze Zahl von 7 bis 21 und X ein Anion bedeuten,
   näher beschrieben werden.

Beispielsweise können die vorstehend erwähnten, bevorzugten Calciumsalze der Verbindung der Formel II (3c) und (3d) bei Vorliegen eines Dicalciumsalzes durch die folgenden Summenformeln näher beschrieben werden, wobei in den Summenformeln X für ein Anion steht:
(3c, 3d/iia) C₅₉H₉₂N₁₄O₁₉Ca₂X₂,
(3c, 3d/iib) C₅₉H₉₃N₁₄O₁₉Ca₂X₃ oder
(3c, 3d/iic) C₅₉H₉₄N₁₄O₁₉Ca₂X₄.

Vorzugsweise ist in sämlichen, vorstehend genannten Summenformeln das Anion X ein Halogenidanion, Cl⁻, Br⁻ oder I⁻, besonders bevorzugt Cl⁻.

Das Monocalciumsalz kann in Abhängigkeit von dem Fettsäuresubstituenten (R1 in Formel II) ferner beispielsweise im Fall einer gesättigten Fettsäure (R1 in Formel II) durch die allgemeine Summenformel

(iii) C₄₆₊ₙH₆₈₊₂ₙN₁₄O₁₉Ca

oder beispielsweise im Fall einer einfach ungesättigten Fettsäure (R1 in Formel II) durch die allgemeine Summenformel

(iv) C₄₆₊ₙH₆₆₊₂ₙN₁₄O₁₉Ca,

wobei n in beiden allgemeinen Summenformeln eine ganze Zahl von 7 bis 21 bedeutet, näher charakterisiert werden.

Beispielsweise können die vorstehend erwähnten, bevorzugten Calciumsalze der Verbindung der Formel II (3c) und (3d) bei Vorliegen eines Monocalciumsalzes durch die folgende Summenformel näher beschrieben werden:

(3c, 3d/iv) C₅₉H₉₂N₁₄O₁₉Ca.

Bei den vorstehend erwähnten, bevorzugten Calciumsalzen der Verbindung der allgemeinen Formel II (3c) und (3d) weist demgegenüber die korrespondierende Säure, nämlich die entsprechende Verbindung der Formel II, beispielsweise die Summenformel C₅₉H₉₄N₁₄O₁₉ auf.

Die im folgenden A 1437-D genannte, zum vorstehend erwähnten, bevorzugten Calciumsalz (3d) korrespondierende Säure (die entsprechende Verbindung der allgemeinen Formel II) weist die nachfolgend dargestellte Struktur auf (Fig. 1):

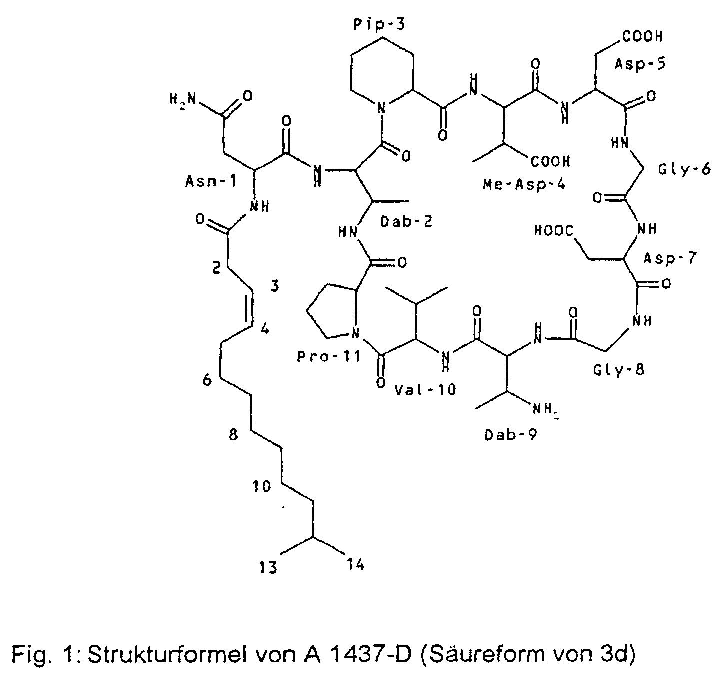

In Tabelle 1 ist die Zuordnung der ¹H und ¹³C-NMR-Signale am Beispiel des A 1437 D Natriumsalzes aufgeführt. Die Aminosäure-Bezeichnungen sind entsprechend den internationalen Konventionen abgekürzt, Dab = 2,3-Diaminobuttersäure, Me-Asp = ß-Methylasparaginsäure, Pip = Pipecolinsäure, FA = Fettsäure. Die Aminosäuren weisen vorzugsweise die folgende Konfiguration auf: Pip-3: D; Me-Asp-4: L-threo; Asp-5, -7: L; Dab-2: L-threo; Dab-9: D-erythro; Val-10: L; Pro-11: L; Asn-1: L.

**Tabelle 1:**

| Chemische Verschiebungen von A 1437-D in CD₃CN/H₂O bei 285 K^{a)}. | | | |
|---|---|---|---|
| AS | Proton/Kohlenstoff | ¹H | ¹³C |
| | | | |
| Asn¹ | NH | 7.82 | - |
| | α | 4.69 | 53.36 |
| | β | 2.70/2.64 | 39.42 |
| | C' | - | 174.59 |
| | β-C' | - | 176.23 |
| | NH₂ | 7.36/6.76 | - |
| Dab² | NH | 7.77 | - |
| | α | 5.04 | 56.49 |
| | β | 4.50 | 48.88 |
| | γ | 1.05 | 20.76 |
| | β-NH | 7.59 | - |
| | C' | - | 172.56 |
| Pip³ | α | 4.64 | 57.39 |
| | β | 2.23/1.68 | 28.86 |
| | γ | 1.64/1.29 | 22.56 |
| | δ | 1.76/1.70 | 26.30 |
| | ε | 3.82/3.36 | 46.41 |
| | C' | - | 175.34 |
| Me-Asp⁴ | NH | 8.93 | - |
| | α | 4.56 | 58.07 |
| | β | 2.51 | 50.6(breit) |
| | γ | 1.05 | 17.24 |
| | C' | - | 176.65 |
| | β-C' | - | 184.13 |
| Asp⁵ | NH | 8.24 | - |
| | α | 4.19 | 57.09 |
| | β | 2.58/2.50 | 40.47 |
| | C' | - | 177.34 |
| | β-C' | - | 179.15 |
| Gly⁶ | NH | 8.83 | - |
| | α | 4.23/3.67 | 45.32 |
| | C' | - | 174.48 |
| Asp⁷ | NH | 7.68 | - |
| | α | 4.57 | 53.85 |
| | β | 3.18/2.44 | 41.93 |
| | C' | - | 176.17 |
| | β-C' | - | 179.21 |
| Gly⁸ | NH | 8.10 | - |
| | α | 4.29/3.55 | 173.23 |
| | C' | - | 173.23 |
| Dab⁹ | NH | 9.54(breit) | - |
| | α | 4.43 | 59.41 |
| | β | 3.54 | 53.45 |
| | γ | 1.34 | 19.78 |
| | C' | - | 172.45 |
| Val¹⁰ | NH | 8.31 | - |
| | α | 4.02 | 62.58 |
| | β | 2.25 | 32.20 |
| | γ | 1.05 | 20.72 |
| | γ' | 0.94 | 21.39 |
| | C' | - | 175.42 |
| Pro¹¹ | α | 4.06 | 63.97 |
| | β | 2.20/1.74 | 32.50 |
| | γ | 1.99/1.91 | 27.91 |
| | δ | 3.83/3.55 | 50.93 |
| | C' | - | 173.57 |
| FA | 1 | - | 175.52 |
| | 2 | 3.03 | 36.84 |
| | 3 | 5.51 | 124.41 |
| | 4 | 5.61 | 136.65 |
| | 5 | 2.05 | 29.82 |
| | 6 | 1.35 | 32.40^{b)} |
| | 7 | 1.25-1.35 | 32.14^{b)} |
| | 8 | 1.25-1.35 | 31.97^{b)} |
| | 9 | 1.25-1.35 | 31.83^{b)} |
| | 10 | 1.29 | 29.96 |
| | 11 | 1.17 | 41.54 |
| | 12 | 1.53 | 30.51 |
| | 13,14 | 0.88 | 24.77 |

| | | | |
|---|---|---|---|
| a) Geeicht wurde auf Natrium-3-(Trimethylsilyl)propionat-2,2,3,3-d₄. | | | |

Es konnte nun beobachtet worden, daß beispielsweise beim Übergang vom A-1437-D-Natriumsalz zum Calciumsalz (3d) die physikochemischen Eigenschaften des Antibiotikums sich grundlegend ändern. So steigt spezifische Drehung [α] des Natriumsalzes bei der Wellenlänge der Natrium-D-Linie und bei 20° C von +6° auf über +52° an, wenn man zu der wässrigen Lösung ein lösliches Calciumsalz, wie z. B. CaCl₂ hinzufügt. Dieses ungewöhnliche Verhalten legt die Annahme nahe, daß das Molekül eine wesentliche Änderung seiner Konformation erfährt. Unterstützt wird diese Konformationsänderung auch durch die geringe Leitfähigkeit des A-1437-D-Calciumsalzes (3d/iv), welche deutlich niedriger ist, als für eine ionogene Verbindung zu erwarten wäre.

Es gibt mehrere Möglichkeiten zur Herstellung von Calciumsalzen der Verbindung der Formel II. Zum einen kann man die begrenzte Löslichkeit der Calciumsalze in ausgewählten Lösungen nutzbar machen. Während die Na⁺- oder die NH₄⁺-Salze in Wasser oder in Methanol sehr leicht löslich und in höheren Alkoholen und anderen polaren organischen Lösungsmitteln löslich sind, sind Löslichkeiten der entsprechenden Calciumsalze in nicht wäßrigen Lösemitteln deutlich herabgesetzt.

Demnach zeichnet sich das Verfahren zur Herstellung des vorstehend beschriebenen Calciumsalzes der Verbindung der Formel II dadurch aus, daß man ein Natrium- oder Ammoniumsalz der Verbindung der Formel II in einem geeignetem organischen Lösungsmittel löst, zu dieser Lösung ein in Ethanol gelöstes Calciumsalz hinzufügt und das Calciumsalz der Verbindung der Formel II als Niederschlag isoliert. Vorzugsweise ist das geeignete organische Lösungsmittel Ethanol. Das in Ethanol gelöste, hinzuzufügende Calciumsalz ist vorzugsweise ein Calciumhalogenid, CaCl₂, CaBr₂, Cal₂ oder die entsprechenden Hydrate. Bei diesem Verfahren entstehen bevorzugt die Dicalciumsalze der Verbindung der Formel II.

Zur Herstellung beispielsweise der Calciumsalze (3d/iia) mit der Summenformel C₅₉H₉₂N₁₄O₁₉Ca₂X₂, (3d/iib) mit der Summenformel C₅₉H₉₃N₁₄O₁₉Ca₂X₃ oder (3d/iic) mit der Summenformel C₅₉H₉₄N₁₄O₁₉Ca₂X₄ kann man deshalb so verfahren, daß man das Natrium- oder Ammonium-Salz von A 1437 D (die zu (3d) korrespondierende Säure) in einem geeigneten organischen Lösemittel, wie z. B. Ethanol löst und zu dieser Lösung ein in Ethanol gelöstes Calciumsalz hinzufügt. Hierbei fällt das im organischen Lösemittel wenig lösliche A 1437 D-Calciumsalz in Form eines Niederschlages aus. Für die Fällung geeignete, in Ethanol lösliche Calciumsalze sind zum Beispiel CaCl₂, CaBr₂ Cal₂ sowie ihre Hydrate. Dieses Fällungsverfahren führt zu Salzen, die neben dem Calciumkation noch Anionen des Fällungssalzes enthalten können, beispielsweise die Halogenide Cl⁻, Br⁻ und I⁻. Die Summenformeln der gefällten Calciumsalze können zum Beispiel lauten: C₅₉H₉₂N₁₄O₁₉Ca₂Halogenid₂, wie z. B. C₅₂H₉₂N₁₄O₁₉Ca₂Cl₂ oder C₅₉H₉₂N₁₄O₁₉Ca₂I₂ oder C₅₉H₉₃N₁₄O₁₉Ca₂Halogenid₃ wie z. B. C₅₉H₉₃N₁₄O₁₉Ca₂Br₃ oder C₅₉H₉₃N₁₄O₁₉Ca₂Cl₃, es können aber auch, je nach Fällungsbedingung, andere Mischsalze entstehen, für die u. a. die Zusammensetzung C₅₉H₉₄N₁₄O₁₉Ca₂Halogenid₄ gefunden wird. Diese gemischten Salze ( A 1437 Calcium-Mischsalze ) sind in Wasser löslich und eignen sich daher zur Behandlung von bakteriellen Infektionen oder zur Konservierung oder auch zur Wachstumsförderung in der Tierzucht, sie können aber auch als Zwischenprodukte für die Herstellung anderer Salze dienen.

Die Kristallisation ist eine weitere Möglichkeit, Calciumsalze der Verbindung der Formel II zu gewinnen oder zu reinigen. Hierbei wird die Eigenschaft des Antibiotikum-Calciumsalzes genutzt, sich in reinem Wasser, in Dimethylsulfoxyd, in reinem Methanol und in anderen polaren Lösungsmitteln zu lösen.

Demgemäß zeichnet sich das Verfahren zur Herstellung eines Calciumsalzes, vorzugsweise des Monocalciumsalzes, der Verbindung der Formel II dadurch aus, daß man ein Dicalciumsalz der Verbindung der Formel II, welches beispielsweise nach der vorstehend beschriebenen Methode gewonnen werden kann, in einem polaren Lösungsmittel löst, anschließend die erhaltene Lösung mit einem weniger polaren Lösungsmittel oder einer Mischung aus weniger polaren Lösungsmitteln versetzt und das Calciumsalz, vorzugsweise das Monocalciumsalz, als Niederschlag isoliert.

Ein weiteres Verfahren zur Herstellung eines Calciumsalzes, vorzugsweise des Monocalciumsalzes, der Verbindung der Formel II zeichnet sich dadurch aus, daß man ein Natrium- oder Ammoniumsalz der Verbindung der Formel II in einem polaren Lösungsmittel löst, zu dieser Lösung ein in demselben Lösungsmittel gelöstes Calciumsalz hinzufügt, anschließend die erhaltene Lösung mit einem weniger polaren Lösungsmittel oder einer weniger polaren Lösungsmittelmischung versetzt und das Calciumsalz, vorzugsweise das Monocalciumsalz, als Niederschlag isoliert.

Vorzugsweise wird bei den beiden Verfahrensalternativen das Natrium- oder Ammoniumsalz oder das Dicalciumsalz der Verbindung der Formel II in einem polaren Lösungsmittel gelöst, das ausgewählt ist aus der Gruppe Wasser, Dimethylsulfoxid und Methanol.

Vorzugsweise ist das weniger polare Lösungsmittel, mit dem die erhaltene Lösung versetzt wird, ausgewählt aus der Gruppe Alkohole, Aceton und Acetonitril.

Vorzugsweise wird das Natrium- oder Ammoniumsalz oder das Dicalciumsalz der Verbindung der Formel II in Wasser gelöst, und das weniger polare Lösungsmittel ist Methanol.

Vorteilhafterweise wird bei beiden Verfahrensvarianten als weniger polare Lösungsmittelmischung ein Gemisch aus Methanol und Butanol zugesetzt.

Wahlweise kann das Calciumsalz, vorzugsweise das Monocalciumsalz, der Verbindung der Formel II dadurch hergestellt werden, daß man ein Natrium- oder Ammoniumsalz der Verbindung der Formel II in Methanol löst, zu dieser Lösung ein in demselben Lösungsmittel gelöstes Calciumsalz hinzufügt, anschließend die erhaltene Lösung mit Wasser oder einer Mischung aus Wasser und Butanol versetzt und das Calciumsalz, vorzugsweise das Monocalciumsalz, als Niederschlag isoliert.

Vorzugsweise fügt man bei sämtlichen dieser Verfahrensvarianten als gelöstes Calciumsalz vorzugsweise ein Calciumhalogenid.hinzu, welches ausgewählt ist aus der Gruppe CaCl₂, CaBr₂, Cal₂ und deren Hydrate.

Beispielsweise kann man das A 1437-D-Dicalciumsalz konzentriert in einem gut lösendem Lösungsmittel lösen und anschließend mit einem mischbaren aber das Antibiotikasalz weniger lösenden Mittel versetzten. Beispiele für letztere sind weniger polare, organische Lösungsmittel, wie Alkohole, Aceton, Acetonitril und weitere. Einen Sonderfall bildet die Mischung aus Wasser und Methanol. Während diese reinen Lösemittel A 1437-D-Ca-Salze leicht lösen, haben die Mischungen aus beiden Lösungsmitteln deutlich schwächere Lösungseigenschaften. Man kann also die A 1437-D-Monocalciumsalze (3d/iv) aus Wasser (aus Methanol) mit Methanol- (Wasser-) Zugabe fällen und kristallisieren. Auf dieser Weise lassen sich Calciumsalze herstellen oder reinigen. Das A 1437-D-Monocalciumsalz (3d/iv) bildet in Wasser-Methanol-Gemischen leicht Gele. Diese Gelbildung ist für die Kristallisation ungünstig, weil dadurch die Kristallisationsgeschwindigkeit stark verlangsamt wird. Für die Kristallisation müssen deshalb Maßnahmen ergriffen werden, um die Gelbildung zu unterdrücken. So eine Maßnahme kann z. B. die Zugabe von geringen Mengen eines geeigneten Stoffes, wie beispielsweise Butanol sein.

Ein anderes Verfahren zur Herstellung des Calciumsalzes der Verbindung der Formel II, vorzugsweise dessen Monocalciumsalzes, beispielsweise das A 1437-D-Monocalciumsalz (3d/iv), besteht in der Verwendung eines Trägers, zum Beispiel von Adsorptionsharzen, Umkehrphasenträgern, Molekularsieben und lonenaustauschern, welcher mit einer wäßrigen Lösung eines Natriun- oder Ammoniumsalzes der Verbindung der Formel II (beispielsweise das Natrium- oder Ammoniumsalz der Verbindung A 1437-D), die mit einem Calciumhalogenid versetzt wurde, oder wahlweise mit einer wäßrigen Lösung eines Dicalciumsalzes der Verbindung der Formel II (beispielsweise die Dicalciumsalze (3d/iia), (3d/iib) oder (3d/iic)) beladen wird, wonach der Träger optional mit einem geeigneten Lösungsmittel gewaschen und schließlich das Calciumsalz der Verbindung der Formel II, vorzugsweise das Monocalciumsalz, beispielsweise das A1437-D-Monocalciumsalz (3d/iv), mit einem geeigneten Lösungsmittel eluiert wird.

Zur Verwendung als Adsorptionsharze sind beispielsweise Amberlite XAD 7 (Rohm & Haas), DIAION® HP20SS (Mitsubishi Chem. Corp.), Poros® 20 R2 oder Polyamid 6 (Riedel-deHaen), bei Verwendung von Umkehrphasen-Trägern sind zum Beispiel LiChrosorb® RP-select B (E. Merck) geeignet. Es können aber auch Träger eingesetzt werden, die gewöhnlich im Rahmen der Hydrophobic Interaction Chromatography (HIC) zum Beispiel für die Proteinreinigung verwendet werden. Solche Träger sind zum Beispiel Phenyl Sepharose® oder TSKgel Phenyl Toyopearl®. Darüber hinaus eignen sich auch Molekularsiebe wie sie für die "size exclusion chromatography" oder Gelfiltrations-Chromatography verwendet werden. Die Grundlage von diesem Verfahren ist die Neigung der Verbindung der Formel II (beispielsweise A1437-D), Calcium-lonen zu binden. Bei der Verwendung von den genannten Trägern wird eine Mischung von Ammonium- oder Natriumsalzen der Verbindung der Formel II mit Calciumsalzen in Wasser hergestellt und diese Mischung in an sich bekannter Weise an den Trägern getrennt. Wahlweise kann auch eine wäßrige Lösung eines entsprechenden Dicalciumsalzes vorgelegt werden. Beispielsweise wird eine wäßrige Lösung von A1437-D-Natriumsalz und Calciumchlorid auf ein Adsorptionsharz, wie zum Beispiel auf DIAION® HP 20SS aufgetragen, das beladene Harz mit Wasser gewaschen, um überschüssige Salze zu entfernen, und anschließend wird das Calciumsalz des Lipopeptids mit wasserhaltigen oder wasserfreien Lösungsmitteln, vorzugsweise mit Methanol von dem Träger eluiert. Die A1437-Calcium-haltigen Fraktionen werden getrocknet. Ein so gewonnenes Produkt hat beispielsweise die Elementzusammensetzung C₅₉H₉₂N₁₄O₁₉Ca (Monocalciumsalz der Verbindung der Formel II (3d/iv)).

Ferner können zur Gewinnung von Calciumsalzen der Verbindung der Formel II Ionenaustauscher, vorzugsweise Anionenaustauscher eingesetzt werden. Bei dieser Methode wird beispielsweise eine beliebige wäßrige, ionenarme Lösung der Verbindung der Formel II bei pH-Werten zwischen pH 5 und pH 9 an einen Anionenaustauscher gebunden, und nach dem Waschen des beladenen Trägers mit Wasser wird das Monocalciumsalz der Verbindung der Formel II mit einer steigenden Konzentration eines in Wasser löslichen Calciumsalzes eluiert. Das Säuleneluat, welches das Monocalciumsalz enthält, wird zum Beispiel durch reverse Osmose entsalzt und getrocknet. Wahlweise kann das Monocalciumsalz durch andere Verfahren, wie z.B. durch Kristallisation isoliert werden.

Die Verbindungen der Formel II, die Vorstufen der erfindungsgemäßen Calciumsalze, lassen sich vorteilhafterweise, wie aus der EP 0 629 636 A1 bekannt, durch Fermentationvon Actinoplanes sp., vorzugsweise Actinoplanes friulensis (DSM 7358), herstellen und wahlweise, wie in der EP 0 688 789 A1 (US 5,629,288) beschrieben, durch Austausch des Säurerestes R1 in Formel II durch einen nicht natürlich vorkommenden Säurerest derivatisieren. Die so gewonnenen Verbindungen der Formel II lassen sich wie vorstehend beschrieben zu deren Calciumsalzen umsetzen.

Die eingangs gestellte Aufgabe, ein verbessertes Verfahren zur fermentativen Herstellung der Verbindung der Formel II, der Säurevorstufe der erfindungsgemäßen Calciumsalze, bereitzustellen, bei dem von dem Mikroorganismus bevorzugt die Verbindung der Formel II produziert wird, wird dadurch gelöst, daß man bei der Fermentation von Actinoplanes spec., vorzugsweise von Actinoplanes friulensis DSM 7358, die Fermentationslösung mit einem oder mehreren Komplexbildnern, vorzugsweise Chelatbildnern, und mit der Aminosäure Asparagin supplementiert.

Vorzugsweise wird als Komplexbildner Zitronensäure oder Ehylendiamintetraacetat (EDTA) verwendet.

Vorteilhafterweise kann auch die Fermentationslösung mit EDTA und Zitronensäure supplementiert werden.

Zur Erhöhung derAusbeute einer Verbindung der Formel II, bei welcher R beispielsweise ein Fettsäurerest der Formel (3a) oder vorzugsweise (3b) ist, kann die Fermentationslösung zusätzlich mit der Aminosäure L-Leucin supplementiert werden.

Zur Erhöhung der Ausbeute an einer Verbindung der Formel II, bei welcher R1 beispielsweise ein Fettsäurerest der Formeln (3c) oder vorzugsweise (3d) bedeutet, kann die Fermentationslösung zusätzlich mit der Aminosäure L-Valin supplementiert werden.

Demgemäß betrifft die vorliegende Erfindung auch ein Verfahren zur Herstellung eines Calciumsalzes der Verbindung der Formel II, das sich dadurch auszeichnet, daß man in einem ersten Schritt die Verbindung der Formel II, wie vorstehend beschrieben, durch Fermentation von Actinoplanes spec., vorzugsweise Actinoplanes friulensis (DSM 7358), herstellt, wobei man die Fermentationslösung mit einem oder mehreren Komplexbildnern, vorzugsweise Chelatbildnern, und mit der Aminosäure Asparagin supplementiert, und man in einem nachfolgenden Schritt das Calciumsalz der Verbindung der Formel II, wie vorstehend erläutert, durch Fällung, Kristallisation oder Behandlung an einem Träger gewinnt.

Die vorliegende Erfindung betirfft auch ein Calciumsalz der Verbindung der Formel II zur Verwendung als Arzneimittel.

Die Calciumsalze der Verbindung der Formel II eignen sich vorzugsweise zur Herstellung eines Arzneimittels gegen bakterielle Infektionen, wobei die vorstehend beschriebenen Calciumsalze (3c) und insbesondere (3d) oder die Dicalciumsalze (3c/iia), (3c/iib) sowie (3c/iic) und insbesondere die Dicalciumsalze (3d/iia), (3d/iib) oder (3d/iic) besonders geeignet sind, wobei das Dicalciumsalz (3d/iia) besonders bevorzugt ist. Besonders eignen sich auch die Monocalciumsalze der Verbindung der Formel II zur Herstellung eines Arzneimittels gegen bakterielle Infektionen, wobei die Monocalciumsalze (3c) und insbesondere (3d) mit der Summenformel (3c, 3d/iv) C₅₉H₉₂N₁₄O₁₉Ca bevorzugt sind. Vorzugsweise eignen sich die vorstehend genannten Calciumsalze der Verbindung der Formel II zur Herstellung eines Arzneimittels gegen bakterielle Infektionen, die von gram-positiven Bakterien, vorzugsweise von glycopeptidresistenten Bakterien, hervorgerufen werden.

Die wenigstens ein Calciumsalz der Verbindung der Formel II enthaltenden Arzneimittel können ferner die üblichen pharmazeutischen Hilfsstoffe enthalten.

Beispielsweise kann das Dicalciumsalz der Verbindung der Formel II, vorzugsweise das Chlorid 3d/iia, zur Herstellung eines Arzneimittels mit gleichen Gewichtsanteilen Mannit in Wasser gelöst und anschließend lyophilisiert werden.

Besonders geeignet sind die Calciumsalze der Verbindung der Formel II aufgrund ihrer Löslichkeit und ihrer toxikologischen Eigenschaften zur parenteralen Verabreichung in Form einer injizierbaren Lösung. Demgemäß betrifft die vorliegende Erfindung auch injizierbare Lösungen, enthaltend ein oder mehrere Calciumsalze der Verbindung der Formel II, vorzugsweise das Calciumsalz (3d/iv) mit der Summenformel C₅₉H₉₂N₁₄O₁₉Ca oder besonders bevorzugt das Calciumsalz 3d/iia mit der Summenformel C₅₉H₉₂N₁₄O₁₉Ca₂Cl₂.

Zur Herstellung einer Injektionslösung kann das aus zu gleichen Gewichtsanteilen an Mannit und Calciumsalz bestehende Lyophilisat in entsprechend aufbereitetem Wasser gelöst werden.

### Beispiele

In den nachfolgenden Beispielen wird die Säureform der Verbindungen der Formel I mit A1437 bezeichnet. Figur 2 gibt eine Übersicht über die in den Beispielen hergestellten bzw. eingesetzten Lipopeptide. Die mit A1437-A, -B und -G bezeichneten Verbindungen der Formel I gehören zu den Lipopeptiden vom Amphomycin-Typ (R¹= OH in Formel I), die mit A1437-C, -D und -H bezeichneten Verbindungen gehören zu den Lipopeptiden vom Asparagin-Typ (R¹= NH₂ in Formel I) bzw. sind Verbindungen der allgemeinen Formel II.

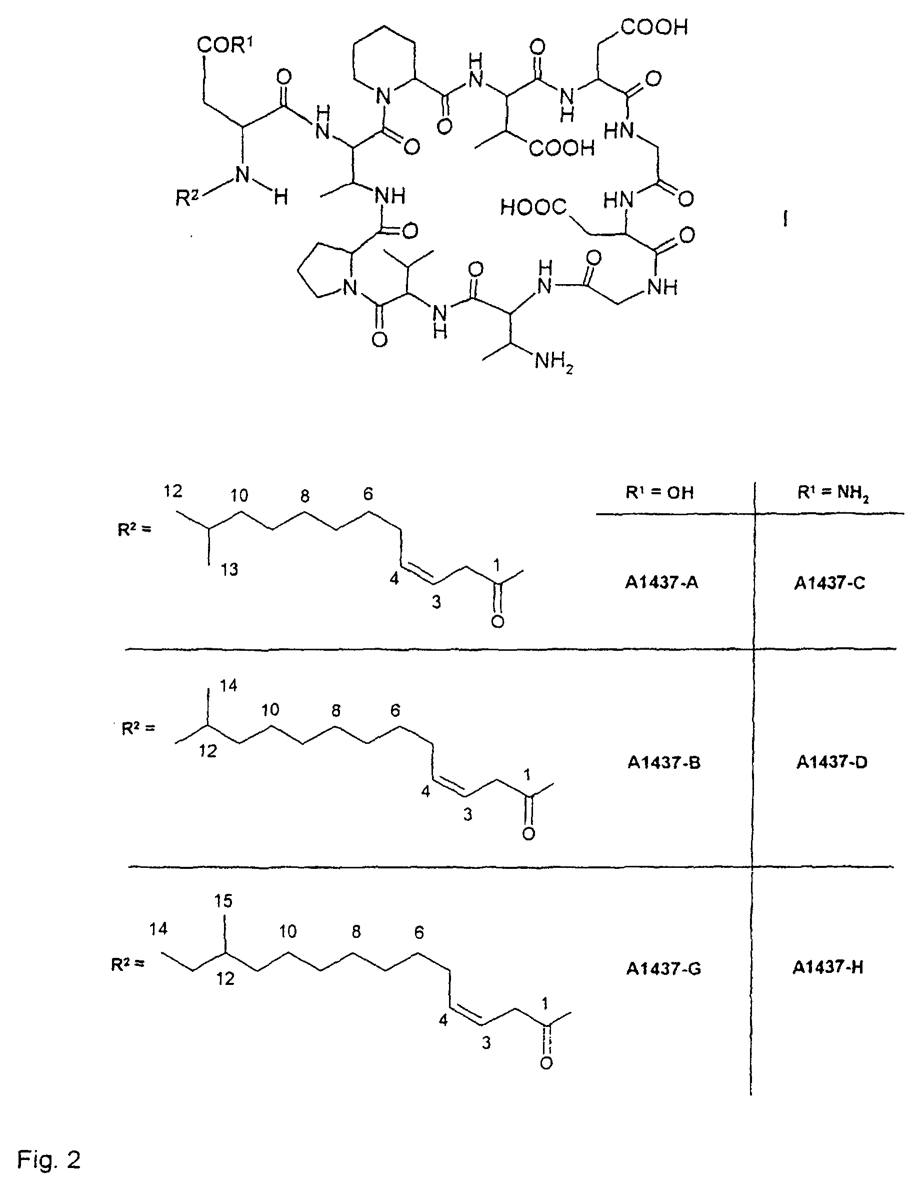

Wie aus der EP 0 629 636 bekannt, bildet Actinoplanes spec., insbesondere Actinoplanes friulensis DSM7358, fermentativ mindestens acht antibiotisch wirksame Verbindungen der Formel I sowohl vom Amphomycin- als auch vom Asparagin-Typ. Am Beispiel der Lipopeptide A1437-C und A1437-D (beides Verbindungen der Formel II) soll gezeigt werden, durch welche Maßnahmen sich die Ausbeuten an Lipopeptiden vom Asparagin-Typ wesentlich erhöhen lassen.

A1437-C und A1437-D sind zyklische Dekapeptide mit einem exozyklischen Asparagin, das im Fall des C-Peptids mit einer C13- und im Fall des D-Peptids mit einer C14-Fettsäure acyliert ist (Fig. 2). Durch Fütterung mit den Aminosäuren Valin bzw. Leucin, die als Starter für die Synthese der jeweiligen Fettsäure dienen, kann die Fermentation in Richtung C- bzw. D-Peptidbildung gesteuert werden. Allerdings werden stets gleichzeitig mit dem C-Peptid ein sog. A-Peptid (A1437-A) und mit dem D-Peptid das B-Peptid (A1437-B) gebildet, das sich vom C- bzw. D-Peptid nur in der exozyklischen Position (Asparaginsäure statt Asparagin) unterscheidet. In der Regel liegen die Titer dieser Peptide sogar noch höher als die des C- und D-Peptids; im günstigsten Fall beträgt das Verhältnis ca. 1:1. Typischerweise bewegten sich die Ausbeuten in der Schüttelkultur zwischen 40 - 150 mg/l D-Peptid bei 50 - 250 mg/l B-Peptid (Beispiel 1). In 30 I und 200 I-Stahlfermentem konnten typischerweise 800 - 1100 mg/l B-Peptid bei 20 - 40 mg/l D-Peptid erzielt werden (Beispiel 2).

Mögliche Ursachen für die in Fermentern reduzierten Ausbeuten an A1437-D sind zum einen mögliche negative Einflüsse von Metallionen, die durch Abrieb vom Fermenter in die Kulturlösung gelangen sowie die die erhöhte Biomassebildung, die zu einem relativen Mangel an Asparagin führt. Um diese Möglichkeiten näher zu untersuchen, wurde die Kulturlösung mit EDTA (0.5 mM) und Zitronensäure (10 mM) als lonenfänger sowie mit Asparagin (0.5 g/l) supplementiert. Hierbei zeigte sich; daß im Stahlfermenter nicht nur die Schüttelkulturausbeute erreicht werden konnte, sondern überraschenderweise auch signifikant übertroffen und das D-Peptid (A1437-D) die prävalente Komponente wurde. Die maximalen Ausbeuten betrugen z.B. 1.2 g/l A1437-D bei 280 mg/l A1437-B (Beispiel 2). Die selektive Verlagerung und Steigerung der Ausbeute in Richtung A1437-D konnte ebenfalls in Schüttelkultur beobachtet werden. Die Ausbeute an A1437-D konnte in Schüttelkultur bei gleicher durchschnittlicher Ausbeute an A1437-B auf ebenfalls 1 g/l gesteigert werden (Beispiel 1).

### Beispiel 1

Erhöhung der Ausbeuten an A1437-D mit Actinoplanes friulensis DSM 7358 in Schüttelkultur durch Zugabe von EDTA und Asparagin

Ein 300 ml-Erlenmeyerkolben mit 100 ml einer Nährlösung (NL 1) folgender Zusammensetzung

| | | |
|---|---|---|
| NL 1 | 30 g/l | Saccharose |
| | 2 g/l | KNO₃ |
| | 1 g/l | K₂HPO₄ |
| | 0,5 g/l | MgSO₄ * 7H₂O |
| | 0,5 g/l | KCl |
| | 0,01 g/l | FeSO₄ * 7H₂O |
| | 2 g/l | Hefeextrakt |
| | 5 g/l | Caseinpepton pH 7.3 |

wird mit einer Ampulle (3 ml Füllmenge) Myzel, das bei -190 °C in flüssigem Stickstoff gelagert war, beimpft und für 5 Tage bei 28 °C und 240 Upm geschüttelt.

Mit 25 ml dieses Kolbens wird ein 2 I-Erlenmeyerkolben, der mit 500 ml der folgenden Nährlösung (NL 2) gefüllt ist, beimpft.

| | | |
|---|---|---|
| NL 2 | 11 | g/l Saccharose |
| | 6 g/l | Fleischextrakt Merck |
| | 0,3 g/l | Hefeextrakt |
| | 0,6 g/l | MgSO₄ * 7H₂O |
| | 0,1 g/l | KH₂PO₄ |
| | 3,9 g/l | L-Valin |
| | 0,0027 g/l | FeCl₃ * 6H₂O pH 7,2 |

Der pH-Wert der Fermentationsbrühe beträgt am Ende der Fermentation 7,0 bis 7,8. Typischerweise lagen die Ausbeuten zwischen 20 und 155 mg/l A1437-D bei 60 -250 mg/l A1437-B.

| Einzelergebnisse | | | |
|---|---|---|---|
| | | A1437-B mg/l | A1437-D mg/l |
| Kolben | 1 | 140 | 85 |
| | 2 | 180 | 140 |
| | 3 | 240 | 155 |
| | 4 | 110 | 130 |
| | 5 | 90 | 20 |
| | 6 | 60 | 40 |

Durch Zugabe von 0,5 mM Ethylen-diamino-tetraessigsäure (EDTA) und 0.5 g/l Asparagin konnten die Ausbeuten an A1437-D auf ca. 1 g/l bei gleicher durchschnittlicher Ausbeute an A1437-B gesteigert werden.

| Einzelergebnisse | | | |
|---|---|---|---|
| | | A1437-B mg/l | A1437-D mg/l |
| Kolben | 1 | 170 | 860 |
| | 2 | 70 | 500 |
| | 3 | 200 | 980 |
| | 4 | 130 | 510 |
| | 5 | 65 | 420 |
| | 6 | 190 | 780 |

### Beispiel 2

Erhöhung der Ausbeuten an A1437-D durch Fermentation von Actinoplanes friulensis DSM 7358 in 200 I-Fermentern durch Zugabe von EDTA und Asparagin.

Ein 300 ml Erlenmeyerkolben mit 100 ml der Nährlösung 1 wird mit dem Inhalt einer bei -190°C gelagerten Ampulle beimpft und für 5 Tage bei 28°C und 240 Upm geschüttelt.

20 ml dieses Kolbens wurden auf einen mit 500 ml derselben Nährlösung gefüllten 2l-Erlenmeyerkolben verimpft, der für ebenfalls 5 Tage bei 28°C und 120 Upm geschüttelt wird.
Der Inhalt von 8 dieser Kolben wird auf einen 40 I-Fermenter verimpft, der mit 30 I der Nährlösung 2 gefüllt ist und für 150 - 250 h bei 28°C, 0,7 vvm und einer Rührergeschwindigkeit von 0,8 m/sec gefahren wird. Der pH-Wert der Fermentationsbrühe beträgt am Ende der Fermentation 7,0 bis 7,8.

Die Ausbeuten liegen typischerweise zwischen 20 und 40 mg/l A1437-D bei einer A1437-B-Konzentration von 800 - 1000 mg/l.

| Einzelergebnisse | | | |
|---|---|---|---|
| | | A1437-B mg/l | A1437-D mg/l |
| Fermenter | 1 | 830 | 20 |
| | 2 | 730 | 40 |
| | 3 | 1050 | 40 |

Durch Zugabe von 0,5 mM EDTA und 0,5 g/l Asparagin konnten die A1437-D-Ausbeuten auf bis zu 1,2 g/l bei erheblicher Reduzierung der A1437-B-Konzentration erhöht werden.

| Einzelergebnisse | | | |
|---|---|---|---|
| | | A1437-B mg/l | A1437-D mg/l |
| Fermenter | 1 | 220 | 690 |
| | 2 | 140 | 930 |
| | 3 | 430 | 1200 |

### Beispiel 3

Ausbeuten an A1437-C durch Fermentation von Actinoplanes friulensis DSM 7358 in Schüttelkultur.

Ein 300 ml-Erlenmeyerkolben mit 100 ml der Nährlösung 1 wird mit dem Inhalt einer bei -190 °C gelagerten Ampulle beimpft und für 5 Tage bei 28 °C und 240 Upm geschüttelt.

Mit 25 ml dieses Kolbens wird ein 2 I-Erlenmeyerkolben, der mit 500 ml der folgenden Nährlösung (NL 3) gefüllt ist, beimpft.

| | | |
|---|---|---|
| NL 3 | 11 g/l | Saccharose |
| | 6 g/l | Fleischextrakt |
| | 0,3 g/l | Hefeextrakt |
| | 0,6 g/l | MgSO₄*7H₂O |
| | 0,1 g/l | KH₂PO₄ |
| | 3,9 g/l | L-Leucin |
| | 0,0027 g/l | FeSO₄ * 7H₂O pH 7.3 |

Der pH-Wert der Fermentationsbrühe beträgt am Ende der Fermentation 7,0 bis 7,8. Die Ausbeuten betragen typischerweise:

| | | A1437-A mg/l | A1437-C mg/l |
|---|---|---|---|
| Kolben | 1 | 166 | 298 |
| | 2 | 543 | 168 |
| | 3 | 518 | 173 |
| | 4 | 435 | 210 |
| | 5 | 345 | 230 |
| | 6 | 225 | 215 |

### Beispiel 4

Tabelle 2 gibt einen Stabilitäts-Vergleich von dem A 1437 D - Natrium-Salz mit dem Calcium-Mischsalz, C₅₉H₉₂N₁₄O₁₉Ca₂I₂, nach Lagerung bei 40° C wieder.

| | Abbaurate des A 1437 D, in %. |
|---|---|
| A 1437-D-Natriumsalz, | 3 % |
| C₅₉H₉₂N₁₄O₁₉Ca₂I₂ : | < 0,5 % |

### Beispiel 5

Die antibakterielle Wirkung des A 1437 D - Ca-Salzes ist in der Tabelle 3 zusammengefaßt. Der Wert der Verbindung ist in ihrer Wirksamkeit gegen die resistenten und multiresistenten Krankheitserreger begründet: das Wirkungsspektrum macht das Antibiotikum - neben der unten aufgeführten guten Verträglichkeit - zu einer Bereicherung des Arzneimittelschatzes.

Tabelle 3: In-Vitro Aktivität des A 1437 D - Ca-Salzes gegen grampositive Bakterien im Reihenverdünnungstest angegeben als minimale Hemm-Konzentration (MHK).

| Keim | Resistenz* | MHK-Werte (µg / ml) |
|---|---|---|
| Staphyloc. aureus, 11HT3 | S | 0.04 |
| Staph. aureus, | ATCC 13709 | 0.6 |
| Staph. aureus, 11HT1 | novR | 0.15 |
| Staph. aureus, 11DU5 | novR, tetR | 0.08 |
| Staph. aureus, 11CB20 | oxaR, eryR, tetR | 0.6 |
| Staph. aureus, 11GO64 | ofIR, oxaR, eryR, novR | 0.6 |
| Staphyloc. coagul. negativ ofIR, | oxaR, tetR | 0.3 |
| Staph. epidermidis, GO20 | tetR | 0.3 |
| Staph. epidermidis, GO42 | oxaR | 0.3 |
| Streptoc. pyogenes, A1SJ1 | eryR | 0.6 |
| Streptoc. pyogenes, A1FI6 | eryR | 0.6 |
| Streptoc. gr. G, GOCB2 | tetR, rifR, novR | 0.3 |
| Streptoc. pneumoniae 30BI2 | eryR | 0.3 |
| Streptoc. milleri, GR12 | S | 0.3 |
| Streptoc. mitis, GR16 | eryR | 0.3 |
| Enteroc. faecium, AP9 | vanR, teiR | 0.6 |
| Enteroc. faecium, HT12 | teiR, vanR, eryR, tetR | 0.6 |
| Enteroc. faecium, IP2 | teiR, vanR, eryR, tetR | 0.6 |
| Enteroc. faecium, HM3 | teiR, vanR, eryR | 0.6 |
| Enteroc. gallinarium, HM8 | vanR, tetR | 0.15 |
| Enteroc. faecalis, HM9 | novR, vanR, eryR | 2.5 |
| Enteroc. faecalis, UC5 | ATCC 29212 novR | 2.5 |
| Enteroc. faecalis, DU18 | tetR, novR | 0.6 |

| | | |
|---|---|---|
| * S = empfindlich, R = resistent, ery = Erythromycin, nov = Novobiocin, ofl = Ofloxacin, oxa = Oxacillin, rif = Rifampicin, tei = Teichopfanin, tet = Tetracyclin, van = Vancomycin. | | |

### Beispiel 6

### Herstellung des A 1437 D-dicalcium-jodid-Salzes (C₅₉H₉₂N₁₄O₁₉Ca₂I₂).

Zu einer Lösung von 1.35 g A 1437 D - Natriumsalz in 27 ml absol. Ethanol werden langsam 0.8 g Cal₂x4H₂O, gelöst in 3 ml Ethanol, gegeben. Hierbei fällt allmählich ein weißer, flockiger Niederschlag aus, der nach 30 Minuten durch Zentrifugieren gesammelt, dreimal mit je 10 ml kaltem Ethylalkohol gewaschen und anschließend im Vakuum getrocknet wird. Es resultieren 1.4 g A 1437 D-dicalcium-jodid-Salz. Die Analysen ergeben neben 79% A 1437D, welches durch HPLC bestimmt wird, 5% Ca, 15.4% Jod und < 0.2% Natrium, entsprechend der Zusammensetzung C₅₉H₉₂N₁₄O₁₉Ca₂I₂.

### Beispiel 7

### Herstellung des A 1437 D-calcium-bromid-Salzes.

18 g A 1437 D - Natriumsalz werden in 360 ml absol. Ethanol gelöst und bei Zimmertemperatur mit 7 g CaBr₂, gelöst in wasserfreiem Ethanol, versetzt. Es fällt hierbei ein weißer, flockiger Niederschlag aus, der durch Zentrifugieren 15 Minuten bei 8000 g gesammelt wird. Zweimaliges Waschen mit je 180 ml absol. Ethanol und anschließendes Trocknen im Vakuum führt zu 18.8 g A 1437 D-calcium-bromid-Salz, dessen Analyse 80% A 1437 D ( HPLC, als freie Säure ), 6% Calcium, 15 % Brom und <0.1% Natrium ergibt.

### Beispiel 8

### Herstellung des A 1437 D-Monocalciumsalzes durch Festphasen-Extraktion.

1,1 g A 1437 D-calcium-bromid-Salz hergestellt entsprechend Beispiel 7 werden in Wasser gelöst und auf eine vorbereitete, 16 ml fassende, mit MCI Gel CHP20P, 75-150µ gefüllte Säule aufgetragen. Eluiert wird, so rasch es geht, mit einem Lösungsmittel-Gradienten von 10% Methanol in Wasser (Lösung A) bis 90% Methanol und 10% Butanol (Lösung B). Es werden zuerst die Verun-reinigungen von der Säule gewaschen, dann mit reiner Lösung B das A 1437 D-Monocalciumsalz. Konzentrieren im Vakuum ergeben 0.8 g A 1437 D-Monocalciumsalz mit 96% A 1437 D ( HPLC ) sowie 3% Calcium, entsprechend der Zusammensetzung C₅₉H₉₂N₁₄O₁₉Ca.

### Beispiel 9

### Umfällen des A 1437 D-calcium-bromid-Salzes.

1 g A 1437 D-Calciumbromid-Salz, gewonnen wie im Beispiel 7 beschrieben, wird in 100 ml Wasser gelöst und mit 36 ml einer Mischung von Methanol-Butanol ( 9:1 ) versetzt. Die zuerst klare Lösung wird allmählich trüb, sie wird zur Vervollständigung 12 Stunden stehengelassen. Durch Zentrifugieren wird der entstandene Niederschlag gesammelt, mit 50 ml kaltem 40%-igem Methanol in Wasser gewaschen und im Vakuum getrocknet. Es werden 720 mg Monocalciumsalz, C₅₉H₉₂N₁₄O₁₉Ca, erhalten mit 3.1 % Calcium, < 1% Brom und 95% A 1437 D-Monocalciumsalzes.

### Beispiel 10

### Gewinnung des A 1437 D-Monocalciumsalzes (C₅₉H₉₂N₁₄O₁₉Ca) durch Methanol-Fällung.

1 g A 1437 D - Natriumsalz werden in 30 ml Wasser gelöst und mit einer Lösung von 200 mg CaCl₂ in 10 ml Wasser versetzt. Zur klaren wässrigen Lösung werden 14 ml einer Mischung von 90% Methanol und 10% Butanol gegeben und nach einer Stunde bei 4° C abzentrifugiert. Der zweimal mit je 30 ml wässrigem Methanol ( 40% ) gewaschene Niederschlag ergibt 810 mg A 1437 D-Monocalciumsalz mit 3.2% Calcium, < 1% Chlorid und 94,5% A 1437 D-Monocalciumsalz, berechnet als freie Säure.

### Beispiel 11

### Bestimmung der in-vitro Hämolyse.

Zur Messung hämolytischen Aktivität wird frisch entnommenes, venöses Blut von Mensch, Rhesusaffen oder Beaglehund verwendet. Das Blut wird in heparinisierten Röhrchen gesammelt und in Aliquots von 200 µl auf 12 Ployethylenröhrchen verteilt. Ein Aliquot wird mit 200 µl destilliertemWasser versetzt und dient als 100 % Standard, ein anderes wird mit 200 µl physiologischer Kochsalzlösung (0.9 % NaCl) gemischt (0% Standard). Je 200 µl von Substanzverdünnungen in physiologischer Kochsalzlösung zu 1600, 800, 400, 200, 100, 50, 25, 12.5, 6.25 und 3.125 mg/l werden auf die übrigen Röhrchen verteilt. Sämtliche Röhrchen werden vorsichtig geschwenkt und dann für 3 Stunden bei 37° C inkubiert. Anschließend wird der 100 % Standard mit 5 ml destilliertem Wasser, die übrigen Röhrchen mit je 5 ml physiologischer Kochsalzlösung aufgefüllt und 5 Minuten bei 700 g zentrifugiert.

Die Hämolyse wird durch Messung der Absorbtion des Überstandes in einem Spektralphotometer bei einer Wellenlänge von 540 nm bestimmt. Die Absorption des Standards mit kompletter Hämolyse wird als 100 % gesetzt. Die Absorption der Testpräparatverdünnungen und des 0 % Standards werden gemessen und als Prozent der maximal induzierbaren Hämolyse angegeben. Die Tabelle 4 zeigt das Ergebnis des Hämolyseversuchs mit A 1437-B, A 1437-G im Vergleich zu A1437-D Ca-Salz, durchgeführt mit Affenblut.

Die Tabelle 4 zeigt beispielhaft die in vitro Hämolyse von Affenblut in Abhängigkeit von den Antobiotika-Konzentrationen.

**Tabelle 4**

| Antibiotika-Konzentrationen (mg/l): | | | | | | | | |
|---|---|---|---|---|---|---|---|---|
| | 12,5 | 25 | 50 | 100 | 200 | 400 | 800 | 1600 |
| A 1437-B | 0 | 2 | 7 | 11 | 16 | 25 | 31 | 39 |
| A 1437-G | 0 | 5 | 8 | 12 | 17 | 25 | 30 | 40 |
| A1437-D Ca-Salz | 0 | 0 | 0 | 3 | 6 | 9 | 15 | 19 |

### Beispiel 12

### Herstellung des A 1437 D-dicalcium-dichlorid-Salzes aus dem Natrium-Salz.

15 g A 1437 D-Natriumsalz werden in 400 ml absolutem Ethanol vollständig gelöst und mit 2,52 CaCl₂ (trocken), gelöst in 100 ml absolutem Ethanol, unter langsamem Rühren während 30 Minuten versetzt und anschließend zwei Stunden bei 0 °C stehen gelassen. Der Niederschlag wird durch Zentrifugieren abgetrennt, mit 200 ml Ethanol gewaschen, erneut abzentrifugiert und im Hochvakuum getrocknet. Die Ausbeute beträgt 16,5 g. Das Endprodukt enthält 80.5% A 1437 D-Lipopeptid, berechnet als freie Säure, 4.5% Wasser, 5.1% Calcium, 7.5% Chlorid und 2.1% Natrium entsprechend der Summenformel C₅₉H₉₂N₁₄O₁₉Ca₂Cl₂ mit einer Beimengung von ca. 4.6% NaCI.

### Beispiel 13

### Kristallisation des A 1437 D-dicalcium-dichlorid-Salzes aus dem Natrium-Salz.

2,5 g A 1437 D-Natriumsalz, Reinheit: 91,9%, werden in 100 ml Wasser gelöst, mit 10 g CaCl₂ ( z. B. Aldrich Cat. No.: 38,314-7) versetzt. Falls hierbei eine Trübung auftritt, wird diese durch Filtration oder durch Zentrifugieren entfernt. Nun wird die Kristallisation durch Zugabe von 35 ml Ethanol im Verlauf von zwei Stunden langsam ausgelöst, wobei bei einem organischen Lösungsmittel-Anteil von 15 % die Kristallbildung allmählich einsetzt. Zur Beschleunigung der Kristallisation kann der Ansatz bei einem Lösungsmittelanteil von 20 % angeimpft werden. Nach beendeter Lösungsmittel-Zugabe läßt man die kristalline Suspension 24 Stunden bei Raumtemperatur stehen, wobei ein gelegentliches Umrühren die Kristallbildung beschleunigt. Zur Vervollständigung der Kristallisation wird der Ansatz über Nacht auf +1° C gekühlt und anschließend abgenutscht. Das aus dichtem Nadelbüscheln bestehende Kristallisat wird mit 5 ml einer kalten Mischung von 25 % Ethanol in Wasser gewaschen und anschließend getrocknet. Es werden 22 g A 1437 D-Dicalciumdichlorid-Salz, entsprechend 81 % Ausbeute, in 97,9 %-iger Reinheit gewonnen. Die Mutterlauge ( 5 g organischer Feststoff, mit etwa 63 %-iger Reinheit ) wird gekühlt stehen gelassen, wobei im Laufe von mehreren Tagen noch weiteres A 1437 D-Dicalciumdichlorid-Salz auskristallisiert.

### Beispiel 14

### Herstellung einer A 1437 D - Dicalciumdichlorid-Injektionslösung

100 mg A 1437 D-Dicalciumdichlorid und 100 mg apyrogenes Mannit werden in 2 ml sterilem Wasser gelöst und lyophilisiert. Das gesamte Lyophilisat, das als Pulver vorliegt, wird in 2 ml Wasser für Injektionslösungen gelöst, in eine sterilisierte Ampulle gefüllt und die Ampulle mit einem Septum verschlossen.

## Patentansprüche

1. Ein Calciumsalz der Verbindung der Formel II 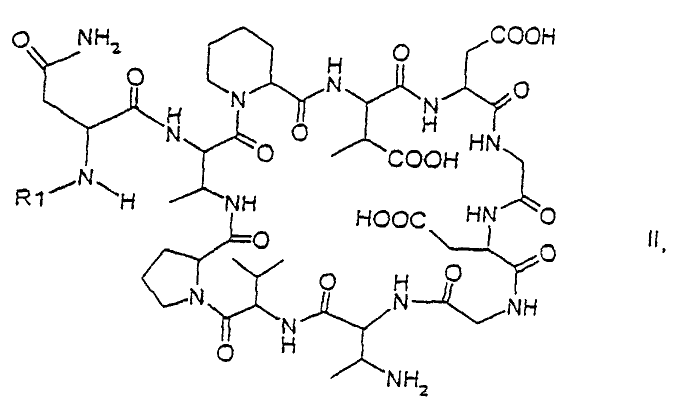 worin R1 ein geradkettiger oder verzweigter, gesättigter oder ungesättigter aliphatischer Acylrest mit 8 bis 22 Kohlenstoffatomen bedeutet, der wahlweise durch ein oder mehrere Phenyl- oder Cycloalkylgruppen unterbrochen oder mit solchen Gruppen verknüpft und farner wahlweise durch Sauerstoff unterbrochen sein kann.

2. Calciumsalz nach Anspruch 1, **dadurch gekennzeichnet, daß** R1 ein durch eine Phenyl- oder Cycloalkylgruppe unterbrochener oder mit einer solchen Gruppe verknüpfter Acylrest bedeutet.

3. Calciumsalz nach Anspruch 2, **dadurch gekennzeichnet, daß** R1 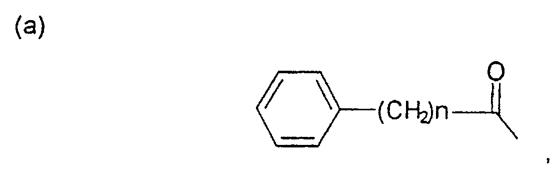 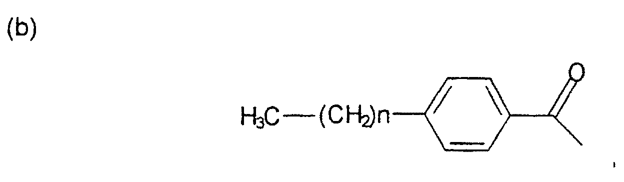 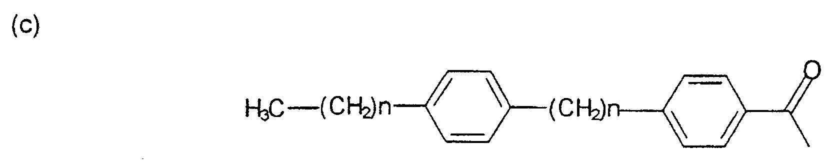 oder 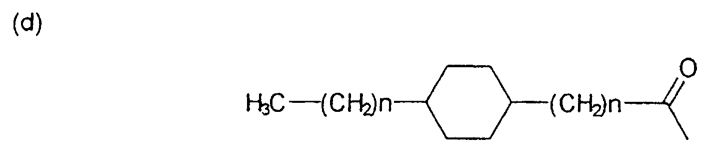 bedeutet, wobei n eine ganze Zahl von 0 bis 20 ist.

4. Calciumsalz nach Anspruch 1, **dadurch gekennzeichnet, daß** R1 ein durch eine Phenyl- oder Cycloalkylgruppe und durch Sauerstoff unterbrochener Acylrest bedeutet.

5. Calciumsalz nach Anspruch 4, **dadurch gekennzeichnet, daß** R1 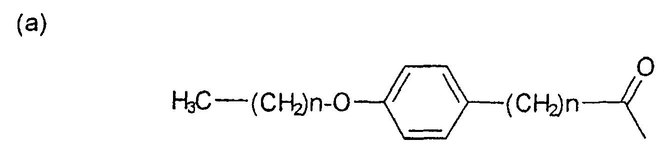 oder 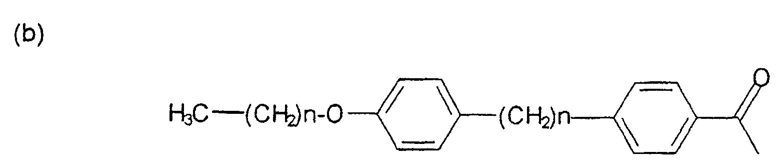 bedeutet, wobei n eine ganze Zahl von 0 bis 20 ist.

6. Calciumsalz nach Anspruch 1, **dadurch gekennzeichnet, daß** R1 ein geradkettiger oder verzweigter, gesättigter oder ungesättigter aliphatischer Acylrest mit 12 bis 15 Kohlenstoffatomen bedeutet.

7. Calciumsalz nach Anspruch 6, **dadurch gekennzeichnet, daß** R1 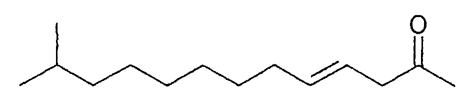 bedeutet.

8. Calciumsalz nach Anspruch 6, **dadurch gekennzeichnet, daß** R1 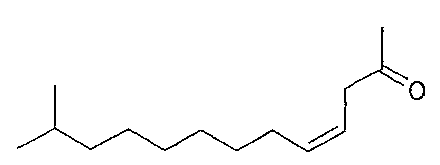 bedeutet.

9. Calciumsalz nach Anspruch 6, **dadurch gekennzeichnet, daß** R1 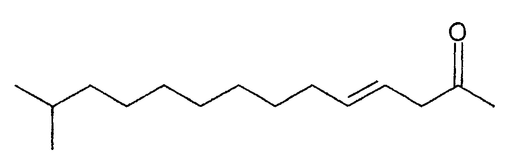 bedeutet.

10. Calciumsalz nach Anspruch 6, **dadurch gekennzeichnet, daß** R1 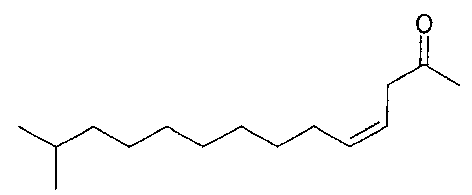 bedeutet.

11. Calciumsalz nach Anspruch 6, **dadurch gekennzeichnet, daß** R1 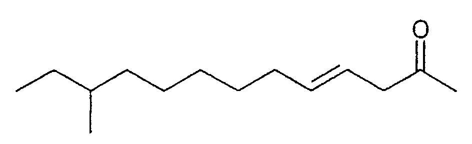 bedeutet.

12. Calciumsalz nach Anspruch 6, **dadurch gekennzeichnet, daß** R1 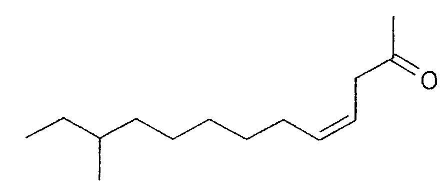 bedeutet.

13. Calciumsalz nach Anspruch 6, **dadurch gekennzeichnet, daß** R1 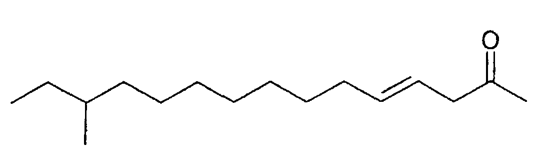 bedeutet.

14. Calciumsalz nach Anspruch 6, **dadurch gekennzeichnet, daß** R1 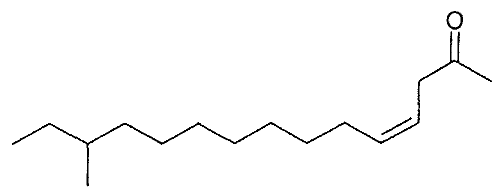 bedeutet.

15. Calciumsalz nach Anspruch 1 oder 6, ferner **gekennzeichnet durch** die allgemeine Summenformel C₄₆₊ₙH₆₈₊₂ₙN₁₄O₁₉Ca₂X₂, worin n eine ganze Zahl von 7 bis 21 und X ein Anion bedeuten.

16. Calciumsalz nach Anspruch 1 oder 6, ferner **gekennzeichnet durch** die allgemeine Summenformel C₄₆₊ₙH₆₉₊₂ₙN₁₄O₁₉Ca₂X₃, worin n eine ganze Zahl von 7 bis 21 und X ein Anion bedeuten.

17. Calciumsalz nach Anspruch 1 oder 6, ferner **gekennzeichnet durch** die allgemeine Summenformel C₄₆₊ₙH₇₀₊₂ₙN₁₄O₁₉Ca₂X₄, worin n eine ganze Zahl von 7 bis 21 und X ein Anion bedeuten.

18. Calciumsalz nach einem oder mehreren der Ansprüche 1, 6 und 7 bis 14, femer **gekennzeichnet durch** die allgemeine Summenformel C₄₆₊ₙH₆₆₊₂ₙN₁₄O₁₉Ca₂X₂, worin n eine ganze Zahl von 7 bis 21 und X ein Anion bedeuten.

19. Calciumsalz nach einem oder mehreren der Ansprüche 1, 6 und 7 bis 14, femer **gekennzeichnet durch** die allgemeine Summenformel C₄₆₊ₙH₆₇₊₂ₙN₁₄O₁₉Ca₂X₃, worin n eine ganze Zahl von 7 bis 21 und X ein Anion bedeuten.

20. Calciumsalz nach einem oder mehreren der Ansprüche 1, 6 und 7 bis 14, ferner **gekennzeichnet durch** die allgemeine Summenformel C₄₆₊ₙH₆₈₊₂ₙN₁₄O₁₉Ca₂X₄, worin n eine ganze Zahl von 7 bis 21 und X ein Anion bedeuten.

21. Calciumsalz nach Anspruch 9 oder 10, ferner **gekennzeichnet durch** die Summenformel C₅₉H₉₂N₁₄O₁₉Ca₂X₂, worin X ein Anion bedeutet.

22. Calciumsalz nach Anspruch 9 oder 10, ferner **gekennzeichnet durch** die Summenformel C₅₉H₉₃N₁₄O₁₉Ca₂X₃, worin X ein Anion bedeutet.

23. Calciumsalz nach Anspruch 9 oder 10, ferner **gekennzeichnet durch** die Summenformel C₅₉H₉₄N₁₄O₁₉Ca₂X₄, worin X ein Anion bedeutet.

24. Calciumsalz nach einem oder mehreren der Ansprüche 15 bis 23, **dadurch gekennzeichnet, daß** X Cl⁻, Br⁻ oder I⁻ bedeutet.

25. Calciumsalz nach Anspruch 24, **dadurch gekennzeichnet, daß** X Cl⁻ bedeutet.

26. Calciumsalz nach Anspruch 1 oder 6, ferner **gekennzeichnet durch** die allgemeine Summenformel C₄₆₊ₙH₆₈₊₂ₙN₁₄O₁₉Ca, worin n eine ganze Zahl von 7 bis 21 bedeutet.

27. Calciumsalz nach einem oder mehreren der Ansprüche 1, 6 und 7 bis 14, ferner **gekennzeichnet durch** die allgemeine Summenformel C₄₆₊ₙH₆₆₊₂ₙN₁₄O₁₉Ca, worin n eine ganze Zahl von 7 bis 21 bedeutet.

28. Calciumsalz nach Anspruch 9 oder 10, ferner **gekennzeichnet durch** die Summenformel C₅₉H₉₂N₁₄O₁₉Ca.

29. Verfahren zur Herstellung eines Calciumsalzes gemäß einem oder mehreren der Ansprüche 1 bis 25, **dadurch gekennzeichnet daß** man ein Natrium- oder Ammoniumsalz der Verbindung der Formel II gemäß einem oder mehreren der Ansprüche 1 bis 14 in einem geeignetem organischen Lösungsmittel löst, zu dieser Lösung ein in Ethanol gelöstes Calciumsalz hinzufügt und das Calciumsalz gemäß einem oder mehreren der Ansprüche 1 bis 25 als Niederschlag isoliert.

30. Verfahren nach Anspruch 29, **dadurch gekennzeichnet, daß** das geeignete organische Lösungsmittel Ethanol ist.

31. Verfahren nach Anspruch 29 oder 30, **dadurch gekennzeichnet, daß** man ein in Ethanol gelöstes Calciumhalogenid, welches ausgewählt ist aus der Gruppe CaCl₂, CaBr₂, CaJ₂ und deren Hydrate, hinzufügt.

32. Verfahren zur Herstellung eines Calciumsalzes gemäß einem oder mehreren der Ansprüche 26 bis 28, **dadurch gekennzeichnet daß** man ein Calciumsalz gemäß einem oder mehreren der Ansprüche 15 bis 25 in einem polaren Lösungsmittel löst, anschließend die erhaltene Lösung mit einem weniger polaren Lösungsmittel oder einer Mischung aus weniger polaren Lösungsmitteln versetzt und das Calciumsalz gemäß einem oder mehreren der Ansprüche 26 bis 28 als Niederschlag isoliert.

33. Verfahren zur Herstellung eines Calciumsalzes gemäß einem oder mehreren der Ansprüche 1 bis 14 und 26 bis 28, **dadurch gekennzeichnet daß** man ein Natrium- oder Ammoniumsalz der Verbindung der Formel II gemäß einem oder mehreren der Ansprüche 1 bis 14 in einem polaren Lösungsmittel löst, zu dieser Lösung ein in demselben Lösungsmittel gelöstes Calciumsalz hinzufügt, anschließend die erhaltene Lösung mit einem weniger polaren Lösungsmittel oder einer weniger polaren Lösungsmittelmischung versetzt und das Calciumsalz gemäß einem oder mehreren der Ansprüche 1 bis 14 und 26 bis 28 als Niederschlag isoliert.

34. Verfahren nach Anspruch 32 oder 33, **dadurch gekennzeichnet, daß** das Natrium- oder Ammoniumsalz der Verbindung der Formel II oder das Calciumsalz gemäß einem oder mehreren der Ansprüche 15 bis 25 in einem polaren Lösungsmittel gelöst wird, das ausgewählt ist aus der Gruppe Wasser, Dimethylsulfoxid und Methanol.

35. Verfahren nach Anspruch 32, 33 oder 34, **dadurch gekennzeichnet, daß** das weniger polare Lösungsmittel ausgewählt ist aus der Gruppe Alkohole, Aceton und Acetonitril.

36. Verfahren nach Anspruch 32 oder einem oder mehreren der Ansprüche 33 bis 35, **dadurch gekennzeichnet, daß** das Natrium- oder Ammoniumsalz der Verbindung der Formel II oder das Calciumsalz gemäß einem oder mehreren der Ansprüche 15 bis 25 in Wasser gelöst wird und das weniger polare Lösungsmittel Methanol ist.

37. Verfahren nach Anspruch 36, **dadurch gekennzeichnet, daß** als weniger polare Lösungsmittelmischung ein Gemisch aus Methanol und Butanol zugesetzt wird.

38. Verfahren zur Herstellung eines Calciumsalzes gemäß einem oder mehreren der Ansprüche 1 bis 14 und 26 bis 28, **dadurch gekennzeichnet daß** man ein Natrium- oder Ammoniumsalz der Verbindung der Formel II gemäß einem oder mehreren der Ansprüche 1 bis 14 in Methanol löst, zu dieser Lösung ein in demselben Lösungsmittel gelöstes Calciumsalz hinzufügt, anschließend die erhaltene Lösung mit Wasser oder einer Mischung aus Wasser und Butanol versetzt und das Calciumsalz gemäß einem oder mehreren der Ansprüche 1 bis 14 und 26 bis 28 als Niederschlag isoliert.

39. Verfahren nach einem oder mehreren der Ansprüche 33 bis 38, **dadurch gekennzeichnet, daß** als gelöstes Calciumsalz ein Calciumhalogenid, welches ausgewählt ist aus der Gruppe CaCl₂, CaBr₂, Cal₂ und deren Hydrate, hinzufügt.

40. Verfahren zur Herstellung eines Calciumsalzes gemäß einem oder mehreren der Ansprüche 26 bis 28, **dadurch gekennzeichnet daß** man eine wäßrige Lösung eines Natrium- oder Ammoniumsalzes der Verbindung der Formel II gemäß einem oder mehreren der Ansprüche 1 bis 14 mit einem Calciumhalogenid versetzt und man diese Lösung oder wahlweise eine wäßrige Lösung eines Calciumsalzes gemäß einem oder mehreren der Ansprüche 15 bis 25 auf einen Träger, der ausgewählt ist aus der Gruppe der Adsorptionsharze, der Umkehrphasenträger, Molekularsiebe und der lonenaustauscher, aufträgt und man schließlich das Calciumsalz gemäß einem oder mehreren der Ansprüche 26 bis 28 mit einem geeigneten Elutionsmittel eluiert.

41. Verfahren zur Herstellung einer Verbindung der Formel II gemäß einem oder mehreren der Ansprüche 1 und 6 bis 14 durch Fermentation von Actinoplanes spec., **dadurch gekennzeichnet, daß** man die Fermentationslösung mit einem oder mehreren Komplexbildnern und mit Asparagin supplementiert.

42. Verfahren nach Anspruch 41, **dadurch gekennzeichnet, daß** der Komplexbildner Zitronensäure ist.

43. Verfahren nach Anspruch 41 oder 42, **dadurch gekennzeichnet, daß** der Komplexbildner EDTA ist.

44. Verfahren nach einem oder mehreren der Ansprüche 41 bis 43, **dadurch gekennzeichnet, daß** die Fermentationslösung mit EDTA und Zitronensäure als Komplexbildnern supplementiert wird.

45. Verfahren nach einem oder mehreren der Ansprüche 41 bis 44 zur Herstellung einer Verbindung der Formel II gemäß Anspruch 7 oder 8, **dadurch gekennzeichnet, daß** die Fermentationslösung ferner mit L-Leucin supplementiert wird.

46. Verfahren nach einem oder mehreren der Ansprüche 41 bis 44 zur Herstellung einer Verbindung der Formel II gemäß Anspruch 9 oder 10, **dadurch gekennzeichnet, daß** die Fermentationslösung ferner mit L-Valin supplementiert wird.

47. Verfahren nach einem oder mehreren der Ansprüche 41 bis 46, **dadurch gekennzeichnet, daß** Actinoplanes friulensis DSM 7358 fermentiert wird.

48. Verfahren zur Herstellung eines Calciumsalzes gemäß einem oder mehreren der Ansprüche 1 und 6 bis 28, **dadurch gekennzeichnet, daß** man zunächst eine Verbindung der Formel II gemäß einem oder mehreren der Ansprüche 1 und 6 bis 14 mit einem Verfahren nach einem oder mehreren der Ansprüche 41 bis 47 herstellt, die Verbindung der Formel II in das Natrium- oder Ammoniumsalz überführt und schließlich das Calciumsalz mit einem Verfahren nach einem oder mehreren der Ansprüche 29 bis 40 gewinnt.

49. Calciumsalz gemäß einem oder mehreren der Ansprüche 1 bis 28 zur Verwendung als Arzneimittel.

50. Calciumsalz gemäß Anspruch 25 zur Verwendung als Arzneimittel.

51. Calciumsalz gemäß Anspruch 28 zur Verwendung als Arzneimittel.

52. Verwendung eines Calciumsalzes gemäß einem oder mehreren der Ansprüche 1 bis 28 zur Herstellung eines Arzneimittels gegen bakterielle Infektionen.

53. Verwendung eines Calciumsalzes gemäß Anspruch 25 zur Herstellung eines Arzneimittels gegen bakterielle Infektionen.

54. Verwendung eines Calciumsalzes gemäß Anspruch 28 zur Herstellung eines Arzneimittels gegen bakterielle Infektionen.

55. Verwendung nach einem der Ansprüche 52 bis 53, **dadurch gekennzeichnet, daß** die bakteriellen Infektionen von gram-positiven Bakterien hervorgerufen werden.

56. Verwendung nach Anspruch 55, **dadurch gekennzeichnet, daß** die gram-positiven Bakterien glykopeptidresistente Bakterien sind.

57. Arzneimittel enthaltend ein oder mehrere Calciumsalze gemäß einem oder mehreren der Ansprüche 1 bis 28 und pharmazeutische Hilfsstoffe.

58. Arzneimittel enthaltend mindestens ein Calciumsalz gemäß Anspruch 25 und pharmazeutische Hilfsstoffe.

59. Arzneimittel enthaltend mindestens ein Calciumsalz gemäß Anspruch 28 und pharmazeutische Hilfsstoffe.

60. Injizierbare Lösung enthaltend ein oder mehrere Calciumsalze gemäß einem oder mehreren der Ansprüche 1 bis 28.

61. Injizierbare Lösung enthaltend mindestens ein Calciumsalz gemäß Anspruch 25.

62. Injizierbare Lösung enthaltend mindestens ein Calciumsalz gemäß Anspruch 28.

## Claims

1. A calcium salt of the compound of the formula II 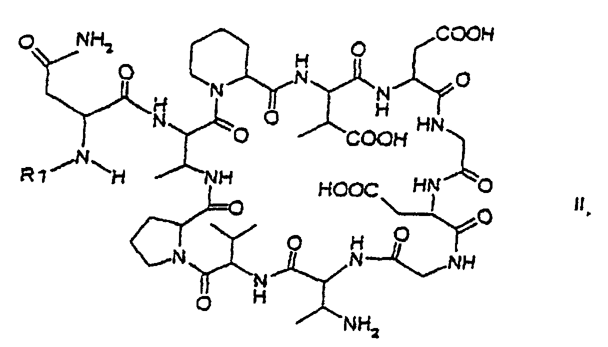 in which R1 is a straight-chain or branched, saturated or unsaturated aliphatic acyl radical having 8 to 22 carbon atoms, which can optionally be interrupted by one or more phenyl or cycloalkyl groups or linked to such groups and can furthermore optionally be interrupted by oxygen.

2. A calcium salt as claimed in claim 1, wherein R1 is an acyl radical interrupted by a phenyl or cycloalkyl group or linked to such a group.

3. A calcium salt as claimed in claim 2, wherein R1 is 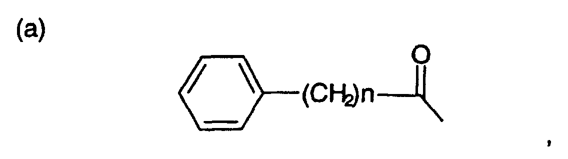 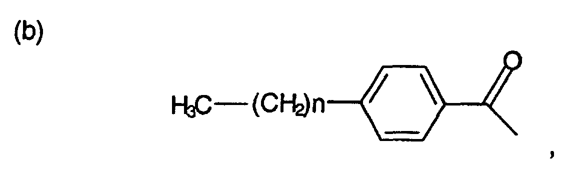 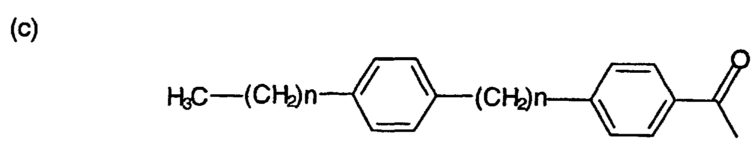 or 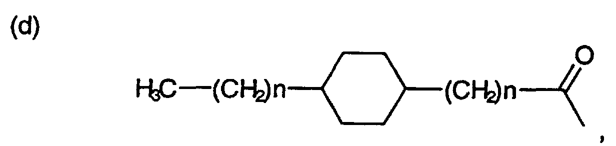 where n is an integer from 0 to 20.

4. A calcium salt as claimed in claim 1, wherein R1 is an acyl radical interrupted by a phenyl or cycloalkyl group and by oxygen.

5. A calcium salt as claimed in claim 4, wherein R1 is 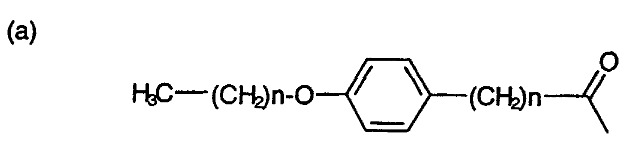 or 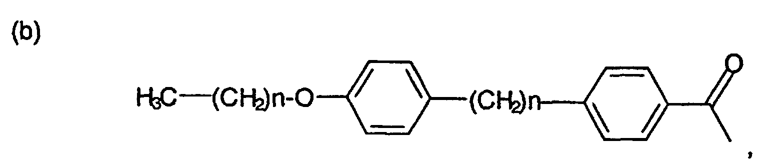 where n is an integer from 0 to 20.

6. A calcium salt as claimed in claim 1, wherein R1 is a straight-chain or branched, saturated or unsaturated aliphatic acyl radical having 12 to 15 carbon atoms.

7. A calcium salt as claimed in claim 6, wherein R1 is

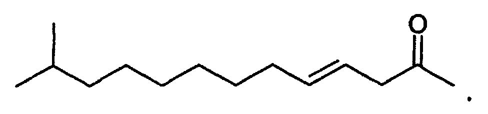

8. A calcium salt as claimed in claim 6, wherein R1 is

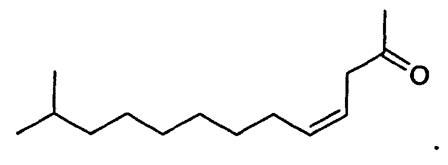

9. A calcium salt as claimed in claim 6, wherein R1 is

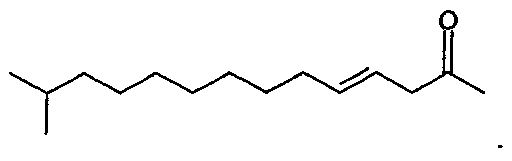

10. A calcium salt as claimed in claim 6, wherein R1 is

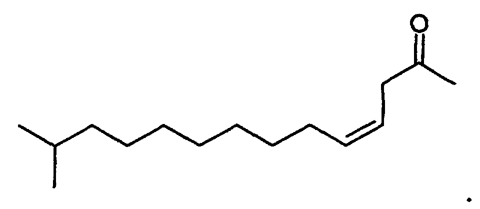

11. A calcium salt as claimed in claim 6, wherein R1 is

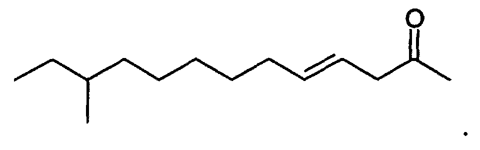

12. A calcium salt as claimed in claim 6, wherein R1 is

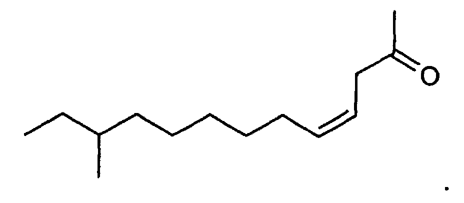

13. A calcium salt as claimed in claim 6, wherein R1 is

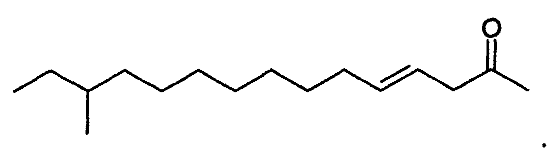

14. A calcium salt as claimed in claim 6, wherein R1 is

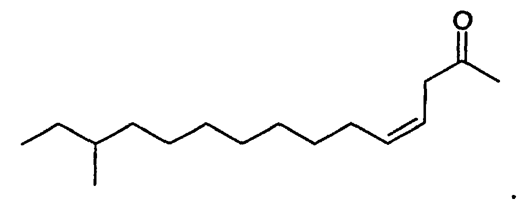

15. A calcium salt according to claim 1 or 6, which furthermore has the empirical formula C₄₆₊ₙH₆₈₊₂ₙN₁₄O₁₉Ca₂X₂, in which n is an integer from 7 to 21 and X is an anion.

16. A calcium salt according to claim 1 or 6, which furthermore has the empirical formula C₄₆₊ₙH₆₉₊₂ₙN₁₄O₁₉Ca₂X₃, in which n is an integer from 7 to 21 and X is an anion.

17. A calcium salt according to claim 1 or 6, which furthermore has the empirical formula C₄₆₊ₙH₇₀₊₂ₙN₁₄O₁₉Ca₂X₄, in which n is an integer from 7 to 21 and X is an anion.

18. A calcium salt as claimed in one or more of claims 1, 6 and 7 to 14, which furthermore has the empirical formula C₄₆₊ₙH₆₆₊₂ₙN₁₄O₁₉Ca₂X₂, in which n is an integer from 7 to 21 and X is an anion.

19. A calcium salt as claimed in one or more of claims 1, 6 and 7 to 14, which furthermore has the empirical formula C₄₆₊ₙH₆₇₊₂ₙN₁₄O₁₉Ca₂X₃, in which n is an integer from 7 to 21 and X is an anion.

20. A calcium salt as claimed in one or more of claims 1, 6 and 7 to 14, which furthermore has the empirical formula C₄₆₊ₙH₆₈₊₂ₙN₁₄O₁₉Ca₂X₄, in which n is an integer from 7 to 21 and X is an anion.

21. A calcium salt as claimed in claim 9 or 10, which furthermore has the empirical formula C₅₉H₉₂N₁₄O₁₉Ca₂X₂, in which X is an anion.

22. A calcium salt as claimed in claim 9 or 10, which furthermore has the empirical formula C₅₉H₉₃N₁₄O₁₉Ca₂X₃, in which X is an anion.

23. A calcium salt as claimed in claim 9 or 10, which furthermore has the empirical formula C₅₉H₉₄N₁₄O₁₉Ca₂X₄, in which X is an anion.

24. A calcium salt as claimed in one or more of claims 15 to 23, wherein X is Cl⁻, Br⁻ or I⁻.

25. A calcium salt as claimed in claim 24, wherein X is Cl⁻.

26. A calcium salt as claimed in claim 1 or 6, which furthermore has the empirical formula C₄₆₊ₙH₆₈₊₂ₙN₁₄O₁₉Ca, in which n is an integer from 7 to 21.

27. A calcium salt as claimed in one or more of claims 1, 6 and 7 to 14, which furthermore has the empirical formula C₄₆₊ₙH₆₆₊₂ₙN₁₄O₁₉Ca, in which n is an integer from 7 to 21.

28. A calcium salt as claimed in claim 9 or 10, which furthermore has the empirical formula C₅₉H₉₂N₁₄O₁₉Ca.

29. A process for the preparation of a calcium salt as claimed in one or more of claims 1 to 25, which comprises dissolving a sodium or ammonium salt of the compound of the formula II as claimed in one or more of claims 1 to 14 in a suitable organic solvent, adding a calcium salt dissolved in ethanol to this solution and isolating the calcium salt as claimed in one or more of claims 1 to 25 as a precipitate.

30. The process as claimed in claim 29, wherein the suitable organic solvent is ethanol.

31. The process as claimed in claim 29 or 30, wherein a calcium halide dissolved in ethanol, which is selected from the group consisting of CaCl₂, CaBr₂, Cal₂ and their hydrates, is added.

32. A process for the preparation of a calcium salt as claimed in one or more of claims 26 to 28, which comprises dissolving a calcium salt as claimed in one or more of claims 15 to 25 in a polar solvent, then treating the solution obtained with a less polar solvent or a mixture of less polar solvents and isolating the calcium salt as claimed in one or more of claims 26 to 28 as a precipitate.

33. A process for the preparation of a calcium salt as claimed in one or more of claims 1 to 14 and 26 to 28, which comprises dissolving a sodium or ammonium salt of the compound of the formula II as claimed in one or more of claims 1 to 14 in a polar solvent, adding a calcium salt dissolved in the same solvent to this solution, then treating the solution obtained with a less polar solvent or a less polar solvent mixture and isolating the calcium salt as claimed in one or more of claims 1 to 14 and 26 to 28 as a precipitate.

34. The process as claimed in claim 32 or 33, wherein the sodium or ammonium salt of the compound of the formula II or the calcium salt as claimed in one or more of claims 15 to 25 is dissolved in a polar solvent which is selected from the group consisting of water, dimethyl sulfoxide and methanol.

35. The process as claimed in claim 32, 33 or 34, wherein the less polar solvent is selected from the group consisting of alcohols, acetone and acetonitrile.

36. The process as claimed in claim 32 or one or more of claims 33 to 35, wherein the sodium or ammonium salt of the compound of the formula II or the calcium salt as claimed in one or more of claims 15 to 25 is dissolved in water and the less polar solvent is methanol.

37. The process as claimed in claim 36, wherein, as a less polar solvent mixture, a mixture of methanol and butanol is added.

38. A process for the preparation of a calcium salt as claimed in one or more of claims 1 to 14 and 26 to 28, which comprises dissolving a sodium or ammonium salt of the compound of the formula II as claimed in one or more of claims 1 to 14 in methanol, adding a calcium salt dissolved in the same solvent to this solution, then treating the solution obtained with water or a mixture of water and butanol and isolating the calcium salt as claimed in one or more of claims 1 to 14 and 26 to 28 as a precipitate.

39. The process as claimed in one or more of claims 33 to 38, wherein, as a dissolved calcium salt, a calcium halide which is selected from the group consisting of CaCl₂, CaBr₂, Cal₂ and their hydrates is added.

40. A process for the preparation of a calcium salt as claimed in one or more of claims 26 to 28, which comprises treating an aqueous solution of a sodium or ammonium salt of the compound of the formula II as claimed in one or more of claims 1 to 14 with a calcium halide and applying this solution or alternatively an aqueous solution of a calcium salt as claimed in one or more of claims 15 to 25 to a support which is selected from the group consisting of the adsorption resins, the reverse-phase supports, molecular sieves and the ion exchangers, and finally eluting the calcium salt as claimed in one or more of claims 26 to 28 using a suitable eluent.

41. A process for the preparation of a compound of the formula II as claimed in one or more of claims 1 and 6 to 14 by fermentation of Actinoplanes spec., which comprises supplementing the fermentation solution with one or more complexing agents and with asparagine.

42. The process as claimed in claim 41, wherein the complexing agent is citric acid.

43. The process as claimed in claim 41 or 42, wherein the complexing agent is EDTA.

44. The process as claimed in one or more of claims 41 to 43, wherein the fermentation solution is supplemented with EDTA and citric acid as complexing agents.

45. The process as claimed in one or more of claims 41 to 44 for the preparation of a compound of the formula II as claimed in claim 7 or 8, wherein the fermentation solution is further supplemented with L-leucine.

46. The process as claimed in one or more of claims 41 to 44 for the preparation of a compound of the formula II as claimed in claim 9 or 10, wherein the fermentation solution is further supplemented with L-valine.

47. The process as claimed in one or more of claims 41 to 46, wherein Actinoplanes friulensis DSM 7358 is fermented.

48. A process for the preparation of a calcium salt as claimed in one or more of claims 1 and 6 to 28, which comprises first preparing a compound of the formula II as claimed in one or more of claims 1 and 6 to 14 using a process as claimed in one or more of claims 41 to 47, converting the compound of the formula II into the sodium or ammonium salt and finally obtaining the calcium salt using a process as claimed in one or more of claims 29 to 40.

49. A calcium salt as claimed in one or more of claims 1 to 28 for use as a pharmaceutical.

50. A calcium salt as claimed in claim 25 for use as a pharmaceutical.

51. A calcium salt as claimed in claim 28 for use as a pharmaceutical.

52. The use of a calcium salt as claimed in one or more of claims 1 to 28 for the production of a pharmaceutical against bacterial infections.

53. The use of a calcium salt as claimed in claim 25 for the production of a pharmaceutical against bacterial infections.

54. The use of a calcium salt as claimed in claim 28 for the production of a pharmaceutical against bacterial infections.

55. The use as claimed in one of claims 52 to 53, wherein the bacterial infections are caused by Gram-positive bacteria.

56. The use as claimed in claim 55, wherein the Gram-positive bacteria are glycopeptide-resistant bacteria.

57. A pharmaceutical comprising one or more calcium salts as claimed in one or more of claims 1 to 28 and pharmaceutical auxiliaries.

58. A pharmaceutical comprising at least one calcium salt as claimed in claim 25 and pharmaceutical auxiliaries.

59. A pharmaceutical comprising at least one calcium salt as claimed in claim 28 and pharmaceutical auxiliaries.

60. An injectable solution comprising one or more calcium salts as claimed in one or more of claims 1 to 28.

61. An injectable solution comprising at least one calcium salt as claimed in claim 25.

62. An injectable solution comprising at least one calcium salt as claimed in claim 28.

## Revendications

1. Sel de calcium du composé de formule II 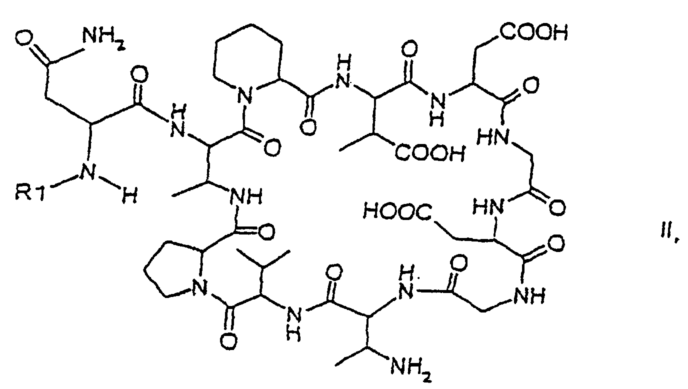 dans laquelle R1 représente un groupe acyle aliphatique, à chaîne linéaire ou ramifiée, saturé ou insaturé, ayant de 8 à 22 atomes de carbone, qui peut être interrompu à volonté par un ou plusieurs groupes phényle ou cycloalkyle ou couplé avec de tels groupes et en outre interrompu à volonté par l'oxygène.

2. Sel de calcium selon la revendication 1, **caractérisé en ce que** R1 représente un groupe acyle interrompu par un groupe phényle ou cycloalkyle ou couplé avec un tel groupe.

3. Sel de calcium selon la revendication 2, **caractérisé en ce que** R1 représente 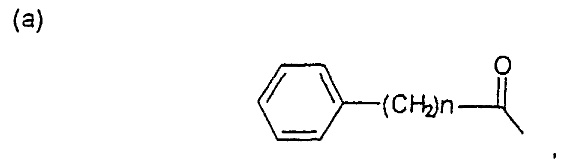 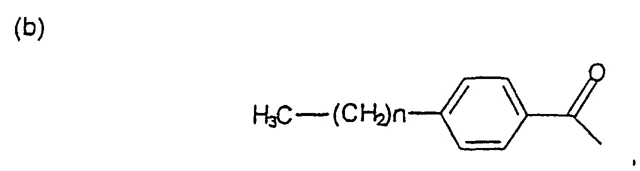 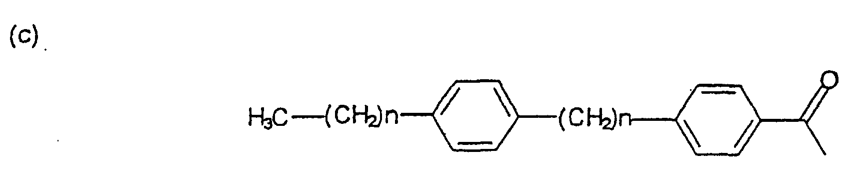 ou 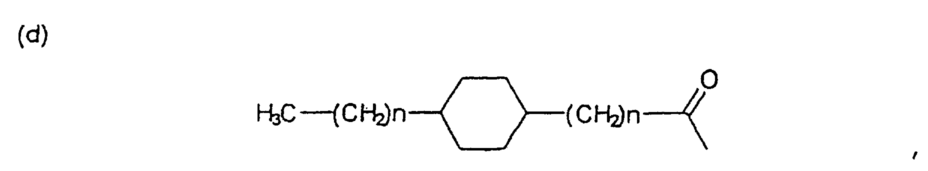 dans laquelle n est un nombre entier de 0 à 20.

4. Sel de calcium selon la revendication 1, **caractérisé en ce que** R1 représente un groupe acyle interrompu par un groupe phényle ou cycloalkyle ou par l'oxygène.

5. Sel de calcium selon la revendication 4, **caractérisé en ce que** R1 représente 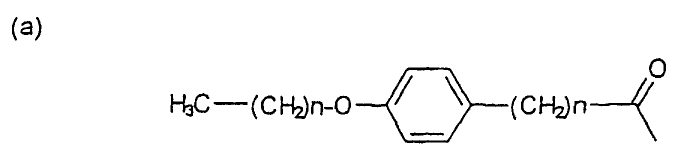 ou 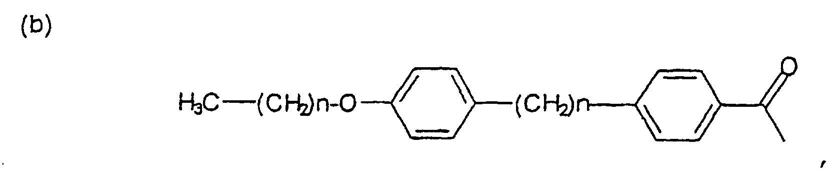 dans laquelle n est un nombre entier de 0 à 20.

6. Sel de calcium selon la revendication 1, **caractérisé en ce que** R1 représente un groupe acyle aliphatique à chaîne linéaire ou ramifiée, saturé ou insaturé ayant de 12 à 15 atomes de carbone.

7. Sel de calcium selon la revendication 6, **caractérisé en ce que** R1 représente

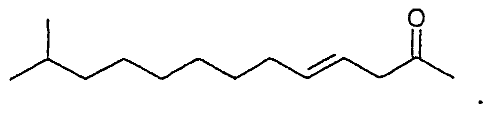

8. Sel de calcium selon la revendication 6, **caractérisé en ce que** R1 représente

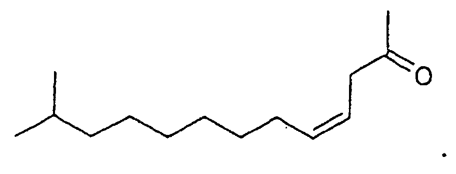

9. Sel de calcium selon la revendication 6, **caractérisé en ce que** R1 représente

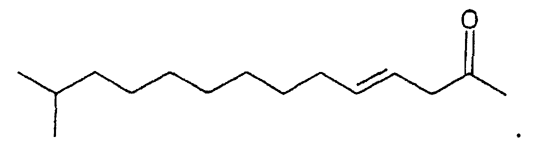

10. Sel de calcium selon la revendication 6, **caractérisé en ce que** R1 représente

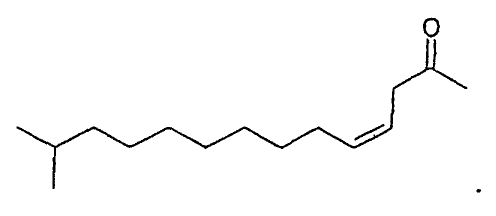

11. Sel de calcium selon la revendication 6, **caractérisé en ce que** R1 représente

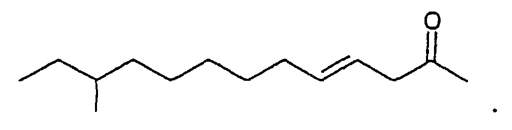

12. Sel de calcium selon la revendication 6, **caractérisé en ce que** R1 représente

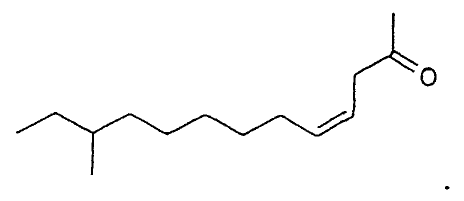

13. Sel de calcium selon la revendication 6, **caractérisé en ce que** R1 représente

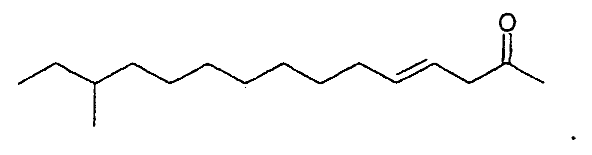

14. Sel de calcium selon la revendication 6, **caractérisé en ce que** R1 représente

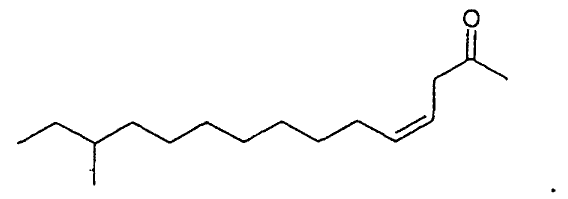

15. Sel de calcium selon la revendication 1 ou 6, **caractérisé en outre par** la formule brute générale C₄₆₊ₙH₆₈₊₂ₙN₁₄O₁₉Ca₂X₂, dans laquelle n représente un nombre entier de 7 à 21 et X un anion.

16. Sel de calcium selon la revendication 1 ou 6, **caractérisé en outre par** la formule brute générale C₄₆₊ₙH₆₉₊₂ₙN₁₄O₁₉Ca₂X₃, dans laquelle n représente un nombre entier de 7 à 21 et X un anion.

17. Sel de calcium selon la revendication 1 ou 6, **caractérisé en outre par** la formule brute générale C₄₆₊ₙH₇₀₊₂ₙN₁₄O₁₉Ca₂X₄, dans laquelle n représente un nombre entier de 7 à 21 et X un anion.

18. Sel de calcium selon une ou plusieurs des revendications 1, 6 et 7 à 14, **caractérisé en outre par** la formule brute générale C₄₆₊ₙH₆₆₊₂ₙN₁₄O₁₉Ca₂X₂, dans laquelle n représente un nombre entier de 7 à 21 et X un anion.

19. Sel de calcium selon une ou plusieurs des revendications 1, 6 et 7 à 14, **caractérisé en outre par** la formule globale générale C₄₆₊ₙH₆₇₊₂ₙN₁₄O₁₉Ca₂X₃, dans laquelle n représente un nombre entier de 7 à 21 et X un anion.

20. Sel de calcium selon une ou plusieurs des revendications 1, 6 et 7 à 14, **caractérisé en outre par** la formule globale générale C₄₆₊ₙH₆₈₊₂ₙN₁₄O₁₉Ca₂X₄, dans laquelle n représente un nombre entier de 7 à 21 et X un anion.

21. Sel de calcium selon la revendication 9 ou 10, **caractérisé en outre par** la formule globale C₅₉H₉₂N₁₄O₁₉Ca₂X₂, dans laquelle X représente un anion.

22. Sel de calcium selon la revendication 9 ou 10, **caractérisé en outre par** la formule globale C₅₉H₉₃N₁₄O₁₉Ca₂X₃, dans laquelle X représente un anion.

23. Sel de calcium selon la revendication 9 ou 10, **caractérisé en outre par** la formule globale C₅₉H₉₄N₁₄O₁₉Ca₂X₄, dans laquelle X représente un anion.

24. Sel de calcium selon une ou plusieurs des revendications 15 à 23, **caractérisé en ce que** X représente Cl⁻, Br⁻ ou I⁻.

25. Sel de calcium selon la revendication 24, **caractérisé en ce que** X représente Cl⁻.

26. Sel de calcium selon la revendication 1 ou 6, **caractérisé en outre par** la formule globale générale C₄₆₊ₙH₆₈₊₂ₙN₁₄O₁₉Ca, dans laquelle n représente un nombre entier de 7 à 21.

27. Sel de calcium selon une ou plusieurs des revendications 1, 6 et 7 à 14, **caractérisé en outre par** la formule globale générale C₄₆₊ₙH₆₆₊₂ₙN₁₄O₁₉Ca, dans laquelle n représente un nombre entier de 7 à 21.

28. Sel de calcium selon la revendication 9 ou 10, **caractérisé en outre par** la formule globale C₅₉H₉₂N₁₄O₁₉Ca.

29. Procédé pour la préparation d'un sel de calcium selon une ou plusieurs des revendications 1 à 25, **caractérisé en ce qu'**on dissout un sel de sodium ou d'ammonium du composé de formule II selon une ou plusieurs des revendications 1 à 14 dans un solvant organique approprié, on ajoute à cette solution un sel de calcium dissous dans l'éthanol et on isole le sel de calcium selon une ou plusieurs des revendications 1 à 25 sous la forme d'un précipité.

30. Procédé selon la revendication 29, **caractérisé en ce que** le solvant organique approprié est l'éthanol.

31. Procédé selon la revendication 29 ou 30, **caractérisé en ce qu'**on ajoute un halogénure de calcium dissous dans l'éthanol, qui est choisi parmi CaCl₂, CaBr₂, CaI₂ et leurs hydrates.

32. Procédé pour la préparation d'un sel de calcium selon une ou plusieurs des revendications 26 à 28, **caractérisé en ce qu'**on dissout un sel de calcium selon une ou plusieurs des revendications 15 à 25 dans un solvant polaire, on ajoute ensuite à la solution obtenue un solvant moins polaire ou un mélange de solvants moins polaires, et on isole le sel de calcium selon une ou plusieurs des revendications 26 à 28 sous la forme d'un précipité.

33. Procédé pour la préparation d'un sel de calcium selon une ou plusieurs des revendications 1 à 14 et 26 à 28, **caractérisé en ce qu'**on dissout un sel de sodium ou d'ammonium du composé de formule II selon une ou plusieurs des revendications 1 à 14 dans un solvant polaire, on ajoute ensuite à cette solution un sel de calcium dissous dans le même solvant, on ajoute ensuite à la solution obtenue un solvant moins polaire ou un mélange de solvants moins polaires, et on isole le sel de calcium selon une ou plusieurs des revendications 1 à 14 et 26 à 28 sous la forme d'un précipité.

34. Procédé selon la revendication 32 ou 33, **caractérisé en ce que** le sel de sodium ou d'ammonium du composé de formule II ou le sel de calcium selon une ou plusieurs des revendications 15 à 25 est dissous dans un solvant polaire, qui est choisi parmi l'eau, le diméthylsulfoxyde et le méthanol.

35. Procédé selon la revendication 32, 33 ou 34, **caractérisé en ce que** le solvant moins polaire est choisi parmi les alcools, l'acétone et l'acétonitrile.

36. Procédé selon la revendication 32 ou une ou plusieurs des revendications 33 à 35, **caractérisé en ce que** le sel de sodium ou d'ammonium du composé de formule II ou le sel de calcium selon une ou plusieurs des revendications 15 à 25 est dissous dans l'eau et le solvant moins polaire est le méthanol.

37. Procédé selon la revendication 36, **caractérisé en ce qu'**on ajoute comme mélange de solvants moins polaires un mélange de méthanol et de butanol.

38. Procédé pour la préparation d'un sel de calcium selon une ou plusieurs des revendications 1 à 14 et 26 à 28, **caractérisé en ce qu'**on dissout un sel de sodium ou d'ammonium du composé de formule II selon une ou plusieurs des revendications 1 à 14 dans le méthanol, on ajoute à cette solution un sel de calcium dissous dans le même solvant, on ajoute ensuite à la solution obtenue de l'eau ou un mélange d'eau et de butanol et on isole le sel de calcium selon un ou plusieurs des revendications 1 à 14 et 26 à 28 sous la forme d'un précipité.

39. Procédé selon une ou plusieurs des revendications 33 à 38, **caractérisé en ce qu'**on ajoute comme sel de calcium dissous un halogénure de calcium, qui est choisi parmi CaCl₂, CaBr₂, CaI₂ et leurs hydrates.

40. Procédé pour la préparation d'un sel de calcium selon une ou plusieurs des revendications 26 à 28, **caractérisé en ce qu'**on ajoute à une solution aqueuse d'un sel de sodium ou d'ammonium du composé de formule II selon une ou plusieurs des revendications 1 à 14 un halogénure de calcium et on dépose cette solution ou à volonté une solution aqueuse d'un sel de calcium selon une ou plusieurs des revendications 15 à 25 sur un support, qui est choisi parmi les résines d'adsorption, les supports d'inversion de phase, les tamis moléculaires et les échangeurs d'ions et on élue finalement le sel de calcium selon une ou plusieurs des revendications 26 à 28 avec un agent d'élution approprié.

41. Procédé pour la préparation d'un composé de formule II selon une ou plusieurs des revendications 1 et 6 à 14 par fermentation de Actinoplanes spec., **caractérisé en ce qu'**on complémente la solution de fermentation avec un ou plusieurs complexants et avec l'asparagine.

42. Procédé selon la revendication 41, **caractérisé en ce que** le complexant est l'acide citrique.

43. Procédé selon la revendication 41 ou 42, **caractérisé en ce que** le complexant est l'EDTA.

44. Procédé selon une ou plusieurs des revendications 41 à 43, **caractérisé en ce que** la solution de fermentation est complémentée avec l'EDTA et l'acide citrique comme complexants.

45. Procédé selon une ou plusieurs des revendications 41 à 44 pour la préparation d'un composé de formule II selon la revendication 7 ou 8, **caractérisé en ce que** la solution de fermentation est complémentée en outre avec la L-leucine.

46. Procédé selon une ou plusieurs des revendications 41 à 44 pour la préparation d'un composé de formule II selon la revendication 9 ou 10, **caractérisé en ce que** la solution de fermentation est complémentée en outre avec la L-valine.

47. Procédé selon une ou plusieurs des revendications 41 à 46, **caractérisé en ce que** Actinoplanes friulensis DSM 7358 est fermenté.

48. Procédé pour la préparation d'un sel de calcium selon une ou plusieurs des revendications 1 et 6 à 28, **caractérisé en ce qu'**on prépare d'abord un composé de formule II selon une ou plusieurs des revendications 1 et 6 à 14 avec un procédé selon une ou plusieurs des revendications 41 à 47, on transforme le composé de formule II en sel de sodium ou d'ammonium et on obtient finalement le sel de calcium avec un procédé selon une ou plusieurs des revendications 29 à 40.

49. Sel de calcium selon une ou plusieurs des revendications 1 à 28 pour l'utilisation comme médicament.

50. Sel de calcium selon la revendication 25 pour l'utilisation comme médicament.

51. Sel de calcium selon la revendication 28 pour l'utilisation comme médicament.

52. Utilisation d'un sel de calcium selon une ou plusieurs des revendications 1 à 28 pour la préparation d'un médicament contre les infections bactériennes.

53. Utilisation d'un sel de calcium selon la revendication 25 pour la préparation d'un médicament contre les infections bactériennes.

54. Utilisation d'un sel de calcium selon la revendication 28 pour la préparation d'un médicament contre les infections bactériennes.

55. Utilisation selon une ou plusieurs des revendications 52 à 53, **caractérisée en ce que** les infections bactériennes sont issues de bactéries à Gramme positif.

56. Utilisation selon la revendication 55, **caractérisée en ce que** les bactéries à Gramme positif sont des bactéries résistances aux glycopeptides.

57. Médicament contenant un ou plusieurs sels de calcium selon une ou plusieurs des revendications 1 à 28 et des substances auxiliaires pharmaceutiques.

58. Médicament contenant qu moins un sel de calcium selon la revendication 25 et des substances auxiliaires pharmaceutiques.

59. Médicament contenant au moins un sel de calcium selon la revendication 28 et des substances auxiliaires pharmaceutiques.

60. Solution injectable contenant un ou plusieurs sels de calcium selon une ou plusieurs des revendications 1 à 28.

61. Solution injectable contenant au moins un sel de calcium selon la revendication 25.

62. Solution injectable contenant au moins un sel de calcium selon la revendication 28.
